# EUROPEAN PATENT APPLICATION

(11) **EP 3 385 276 A1**
(43) Date of publication of application: **10.10.2018**
(21) Application number: 17305400.8
(22) Date of filing: 03.04.2017
(51) Int. Cl.: C07K 14/245, A61K 38/16

(54) **PROTEINS DERIVED FROM CLPB AND USES THEREOF**

(71) Applicant: Targedys, 76000 Rouen (FR); INSERM (Institut National de la Santé et de la Recherche Médicale), 75654 Paris Cedex 13 (FR); UNIVERSITE DE ROUEN, 76130 Mont-Saint-Aignan (FR)
(72) Inventor: FETISSOV, Serguei, 76380 Montigny (FR); LAMBERT, Grégory, 92290 Châtenay-Malabry (FR); LEGRAND, Romain, 76800 Saint Étienne du Rouvray (FR); LUCAS, Nicolas, 76000 Rouen (FR)
(74) Representative: Icosa

(57) **Abstract**

The present invention relates to polypeptides and proteins comprising a fragment of a ClpB protein and compositions therefrom. The present invention also relates to the treatment and/or prevention of inflammation, in particular overweight and/or obesity-related diseases and disorders. The present invention also relates to methods of inducing satiation, prolonging satiety, reducing meal size, reducing food intake, controlling weight gain and stimulating weight loss.

SEQUENCE LISTING

<110> TARGEDYS
      INSTITUT NATIONAL DE LA SANT\311 ET DE LA RECHERCHE M\311DICALE
(INSERM)
      UNIVERSIT\311 DE ROUEN
      FETISSOV Serguei
      LAMBERT Gr\351gory
      LEGRAND Romain
      LUCAS Nicolas

<120> PROTEINS DERIVED FROM CLPB AND USES THEREOF

<130> CDM - 754/EP

<160> 21

<170> BiSSAP 1.3.6

<210> 1
<211> 857
<212> PRT
<213> Hafnia alvei

<220>
<223> ClpB

<400> 1
Met Arg Leu Asp Arg Leu Thr Ser Lys Phe Gln Leu Ala Leu Ala Asp
1               5                   10                  15
Ala Gln Ser Leu Ala Leu Gly Arg Asp Asn Gln Phe Ile Glu Pro Val
            20                  25                  30
His Leu Met Ser Ala Leu Leu Asn Gln Asp Gly Gly Thr Val Arg Pro
        35                  40                  45
Leu Leu Thr Ser Ala Gly Val Asn Thr Ala Ser Leu Arg Gln Glu Leu
    50                  55                  60
Glu Gln Ala Ile Ser Arg Leu Pro Gln Val Glu Gly Ala Gly Gly Asp
65                  70                  75                  80
Val Gln Pro Ser Asn Glu Leu Ile Arg Val Leu Asn Leu Cys Asp Lys
                85                  90                  95
Leu Ala Gln Lys Arg Asn Asp Thr Phe Ile Ser Ser Glu Leu Phe Val
            100                 105                 110
Leu Ala Val Leu Glu Asp Arg Gly Asn Leu Gly Asp Ile Leu Lys Ala
        115                 120                 125
Ala Gly Ala Asn Val Gln Lys Val Ser Thr Ala Ile Glu Gln Met Arg
    130                 135                 140
Gly Gly Glu Lys Val Asp Asp Ala Asn Ala Glu Asp Gln Arg Gln Ala
145                 150                 155                 160
Leu Lys Lys Tyr Thr Val Asp Leu Thr Glu Arg Ala Glu Gln Gly Lys
                165                 170                 175
Leu Asp Pro Val Ile Gly Arg Asp Glu Glu Ile Arg Arg Thr Ile Gln
            180                 185                 190
Val Leu Gln Arg Arg Thr Lys Asn Asn Pro Val Leu Ile Gly Glu Pro
        195                 200                 205
Gly Val Gly Lys Thr Ala Ile Val Glu Gly Leu Ala Gln Arg Ile Ile
    210                 215                 220
Asn Gly Glu Val Pro Glu Gly Leu Lys Asn Lys Arg Val Leu Ser Leu
225                 230                 235                 240
Asp Met Gly Ser Leu Ile Ala Gly Ala Lys Phe Arg Gly Glu Phe Glu
                245                 250                 255
Glu Arg Leu Lys Ala Val Leu Ser Asp Leu Ser Lys Gln Glu Gly Asn
            260                 265                 270

Val Ile Leu Phe Ile Asp Glu Leu His Thr Met Val Gly Ala Gly Lys
        275                 280                 285
Gly Asp Gly Ala Met Asp Ala Gly Asn Met Leu Lys Pro Ala Leu Ala
    290                 295                 300
Arg Gly Glu Leu His Cys Val Gly Ala Thr Thr Leu Asp Glu Tyr Arg
305                 310                 315                 320
Gln Tyr Ile Glu Lys Asp Ala Ala Leu Glu Arg Arg Phe Gln Lys Val
                325                 330                 335
Phe Val Ala Glu Pro Thr Val Glu Asp Thr Ile Ala Ile Leu Arg Gly
            340                 345                 350
Leu Lys Glu Arg Tyr Glu Leu His His His Val Gln Ile Thr Asp Pro
        355                 360                 365
Ala Ile Val Ala Ala Ala Asn Leu Ser His Arg Tyr Ile Ala Asp Arg
    370                 375                 380
Gln Leu Pro Asp Lys Ala Ile Asp Leu Ile Asp Glu Ala Ala Ser Ser
385                 390                 395                 400
Ile Arg Met Gln Met Asp Ser Lys Pro Glu Ser Leu Asp Arg Leu Glu
                405                 410                 415
Arg Arg Ile Ile Gln Leu Lys Leu Glu Gln Gln Ala Leu Asn Lys Glu
            420                 425                 430
Ser Asp Glu Ala Ser Lys Lys Arg Leu Glu Met Leu Asn Glu Glu Leu
        435                 440                 445
Glu His Lys Glu Arg Glu Tyr Ser Glu Leu Glu Glu Glu Trp Lys Ala
    450                 455                 460
Glu Lys Ala Ser Leu Ser Gly Thr Gln Asn Ile Lys Ala Glu Ile Glu
465                 470                 475                 480
Gln Ala Lys Ile Ala Leu Glu Gln Ala Arg Arg Val Gly Asp Leu Ala
                485                 490                 495
Lys Met Ser Glu Ile Gln Tyr Gly Lys Leu Pro Gly Leu Glu Lys Gln
            500                 505                 510
Leu Glu Ala Ala Thr Gln Ser Glu Gly Lys Thr Met Lys Leu Leu Arg
        515                 520                 525
Asn Lys Val Thr Asp Val Glu Ile Ala Glu Val Leu Ala Arg Trp Thr
    530                 535                 540
Gly Ile Pro Val Ser Arg Met Leu Glu Ser Glu Arg Ala Lys Leu Leu
545                 550                 555                 560
Arg Met Glu Asp Asp Leu His Glu Arg Val Ile Gly Gln Asn Glu Ala
                565                 570                 575
Val Glu Ala Val Ser Asn Ala Ile Arg Arg Ser Arg Ala Gly Leu Ser
            580                 585                 590
Asp Pro Asn Arg Pro Ile Gly Ser Phe Leu Phe Leu Gly Pro Thr Gly
        595                 600                 605
Val Gly Lys Thr Glu Leu Cys Lys Ala Leu Ala Asn Phe Leu Phe Asp
    610                 615                 620
Ser Asp Asp Ala Met Val Arg Ile Asp Met Ser Glu Phe Met Glu Lys
625                 630                 635                 640
His Ser Val Ser Arg Leu Val Gly Ala Pro Pro Gly Tyr Val Gly Tyr
                645                 650                 655
Glu Glu Gly Gly Tyr Leu Thr Glu Ala Val Arg Arg Arg Pro Tyr Ser
            660                 665                 670
Val Ile Leu Leu Asp Glu Val Glu Lys Ala His Pro Asp Val Phe Asn
        675                 680                 685
Ile Leu Leu Gln Val Leu Asp Asp Gly Arg Leu Thr Asp Gly Gln Gly
    690                 695                 700
Arg Thr Val Asp Phe Arg Asn Thr Val Val Ile Met Thr Ser Asn Leu
705                 710                 715                 720
Gly Ser Asp Leu Ile Gln Asp Arg Phe Gly Gln Leu Asp Tyr Gly Glu
                725                 730                 735
Met Lys Glu Leu Val Met Ser Val Val Ser His His Phe Arg Pro Glu
            740                 745                 750
Phe Ile Asn Arg Ile Asp Glu Thr Val Val Phe His Pro Leu Asp Gln
        755                 760                 765
Lys Asn Ile Lys Ala Ile Ala Arg Ile Gln Leu Glu Arg Leu Tyr Gln

    770                 775                 780
Arg Leu Glu Glu Lys Gly Tyr Ser Val Thr Ile Thr Asp Ala Ala Leu
785                 790                 795                 800
Glu Gln Leu Ser Lys Ala Gly Phe Asp Pro Val Tyr Gly Ala Arg Pro
                805                 810                 815
Leu Lys Arg Ala Ile Gln Gln Glu Ile Glu Asn Pro Leu Ala Gln Gln
            820                 825                 830
Ile Leu Ser Gly Lys Leu Ile Pro Gly Lys Asp Ile Thr Leu Asp Val
        835                 840                 845
Ala Asp Glu Gln Ile Val Ala Lys Gln
    850                 855

<210> 2
<211> 4
<212> PRT
<213> Artificial Sequence

<220>
<223> ClpB fragment

<220>
<221> SITE
<222> 1
<223> X is E or D

<220>
<221> SITE
<222> 2
<223> X is 2, 3 or 4 of any amino acid

<400> 2
Xaa Xaa Arg Trp
1

<210> 3
<211> 6
<212> PRT
<213> Artificial Sequence

<220>
<223> ClpB fragment

<220>
<221> SITE
<222> 1
<223> X is E or D

<220>
<221> SITE
<222> 3
<223> X is any amino acid

<220>
<221> SITE
<222> 4
<223> X is any amino acid

<400> 3
Xaa Val Xaa Xaa Arg Trp
1               5

<210> 4
<211> 6
<212> PRT
<213> Artificial Sequence

<220>
<223> ClpB fragment

<220>
<221> SITE
<222> 1
<223> X is E or D

<400> 4
Xaa Val Leu Ala Arg Trp
1               5

<210> 5
<211> 6
<212> PRT
<213> Artificial Sequence

<220>
<223> ClpB fragment

<400> 5
Glu Val Leu Ala Arg Trp
1               5

<210> 6
<211> 9
<212> PRT
<213> Artificial Sequence

<220>
<223> ClpB fragment

<220>
<221> SITE
<222> 1
<223> X is E or D

<220>
<221> SITE
<222> 2
<223> X is 2, 3 or 4 of any amino acid

<220>
<221> SITE
<222> 5
<223> X is any amino acid

<220>
<221> SITE
<222> 7
<223> X is any amino acid

<400> 6
Xaa Xaa Arg Trp Xaa Gly Xaa Pro Val
1               5

<210> 7
<211> 9
<212> PRT
<213> Artificial Sequence

<220>
<223> ClpB fragment

<220>
<221> SITE
<222> 1
<223> X is E or D

<220>
<221> SITE
<222> 2
<223> X is 2, 3 or 4 of any amino acid

<220>
<221> SITE
<222> 5
<223> X is any amino acid

<220>
<221> SITE
<222> 7
<223> X is I or K

<400> 7
Xaa Xaa Arg Trp Xaa Gly Xaa Pro Val
1               5

<210> 8
<211> 9
<212> PRT
<213> Artificial Sequence

<220>
<223> ClpB fragment

<220>
<221> SITE
<222> 2
<223> X is 2, 3 or 4 of any amino acid

<220>
<221> SITE
<222> 5
<223> X is any amino acid

<220>
<221> SITE
<222> 7
<223> X is I or K

<400> 8
Glu Xaa Arg Trp Xaa Gly Xaa Pro Val
1               5

<210> 9

<211> 9
<212> PRT
<213> Artificial Sequence

<220>
<223> ClpB fragment

<220>
<221> SITE
<222> 2
<223> X is 2, 3 or 4 of any amino acid

<220>
<221> SITE
<222> 5
<223> X is any amino acid

<400> 9
Glu Xaa Arg Trp Xaa Gly Ile Pro Val
1               5

<210> 10
<211> 9
<212> PRT
<213> Artificial Sequence

<220>
<223> ClpB fragment

<220>
<221> SITE
<222> 2
<223> X is 2, 3 or 4 of any amino acid

<400> 10
Glu Xaa Arg Trp Thr Gly Ile Pro Val
1               5

<210> 11
<211> 11
<212> PRT
<213> Artificial Sequence

<220>
<223> ClpB fragment

<220>
<221> SITE
<222> 3
<223> X is any amino acid

<220>
<221> SITE
<222> 4
<223> X is any amino acid

<400> 11
Glu Val Xaa Xaa Arg Trp Thr Gly Ile Pro Val
1               5                   10

<210> 12
<211> 11
<212> PRT
<213> Artificial Sequence

<220>
<223> ClpB fragment

<400> 12
Glu Val Leu Ala Arg Trp Thr Gly Ile Pro Val
1               5                   10

<210> 13
<211> 9
<212> PRT
<213> Artificial Sequence

<220>
<223> ClpB fragment

<220>
<221> SITE
<222> 2
<223> X is 2, 3 or 4 of any amino acid

<220>
<221> SITE
<222> 5
<223> X is any amino acid

<400> 13
Glu Xaa Arg Trp Xaa Gly Lys Pro Val
1               5

<210> 14
<211> 9
<212> PRT
<213> Artificial Sequence

<220>
<223> ClpB fragment

<220>
<221> SITE
<222> 2
<223> X is 2, 3 or 4 of any amino acid

<400> 14
Glu Xaa Arg Trp Thr Gly Lys Pro Val
1               5

<210> 15
<211> 11
<212> PRT
<213> Artificial Sequence

<220>
<223> ClpB fragment

<220>
<221> SITE
<222> 3
<223> X is amino acid

<220>
<221> SITE
<222> 4
<223> X is any amino acid

<400> 15
Glu Val Xaa Xaa Arg Trp Thr Gly Lys Pro Val
1               5                   10

<210> 16
<211> 11
<212> PRT
<213> Artificial Sequence


<220>
<223> ClpB fragment

<400> 16
Glu Val Leu Ala Arg Trp Thr Gly Lys Pro Val
1               5                   10

<210> 17
<211> 11
<212> PRT
<213> Artificial Sequence


<220>
<223> ClpB fragment

<400> 17
Glu Val Leu Ala Arg Trp Thr Gly Ile Pro Val
1               5                   10

<210> 18
<211> 13
<212> PRT
<213> Artificial Sequence


<220>
<223> ClpB fragment

<400> 18
Ile Ala Glu Val Leu Ala Arg Trp Thr Gly Ile Pro Val
1               5                   10

<210> 19
<211> 15
<212> PRT
<213> Artificial Sequence


<220>
<223> ClpB fragment

<400> 19

Ala Glu Ile Ala Glu Val Leu Ala Arg Trp Thr Gly Ile Pro Val
1               5                   10                  15

<210> 20
<211> 13
<212> PRT
<213> Homo sapiens

<220>
<223> Alpha-MSH

<400> 20
Ser Tyr Ser Met Glu His Phe Arg Trp Gly Lys Pro Val
1               5                   10

<210> 21
<211> 857
<212> PRT
<213> Escherichia coli

<220>
<223> ClpB

<400> 21
Met Arg Leu Asp Arg Leu Thr Asn Lys Phe Gln Leu Ala Leu Ala Asp
1               5                   10                  15
Ala Gln Ser Leu Ala Leu Gly His Asp Asn Gln Phe Ile Glu Pro Leu
            20                  25                  30
His Leu Met Ser Ala Leu Leu Asn Gln Glu Gly Gly Ser Val Ser Pro
        35                  40                  45
Leu Leu Thr Ser Ala Gly Ile Asn Ala Gly Gln Leu Arg Thr Asp Ile
    50                  55                  60
Asn Gln Ala Leu Asn Arg Leu Pro Gln Val Glu Gly Thr Gly Gly Asp
65                  70                  75                  80
Val Gln Pro Ser Gln Asp Leu Val Arg Val Leu Asn Leu Cys Asp Lys
                85                  90                  95
Leu Ala Gln Lys Arg Gly Asp Asn Phe Ile Ser Ser Glu Leu Phe Val
            100                 105                 110
Leu Ala Ala Leu Glu Ser Arg Gly Thr Leu Ala Asp Ile Leu Lys Ala
        115                 120                 125
Ala Gly Ala Thr Thr Ala Asn Ile Thr Gln Ala Ile Glu Gln Met Arg
    130                 135                 140
Gly Gly Glu Ser Val Asn Asp Gln Gly Ala Glu Asp Gln Arg Gln Ala
145                 150                 155                 160
Leu Lys Lys Tyr Thr Ile Asp Leu Thr Glu Arg Ala Glu Gln Gly Lys
                165                 170                 175
Leu Asp Pro Val Ile Gly Arg Asp Glu Glu Ile Arg Arg Thr Ile Gln
            180                 185                 190
Val Leu Gln Arg Arg Thr Lys Asn Asn Pro Val Leu Ile Gly Glu Pro
        195                 200                 205
Gly Val Gly Lys Thr Ala Ile Val Glu Gly Leu Ala Gln Arg Ile Ile
    210                 215                 220
Asn Gly Glu Val Pro Glu Gly Leu Lys Gly Arg Arg Val Leu Ala Leu
225                 230                 235                 240
Asp Met Gly Ala Leu Val Ala Gly Ala Lys Tyr Arg Gly Glu Phe Glu
                245                 250                 255
Glu Arg Leu Lys Gly Val Leu Asn Asp Leu Ala Lys Gln Glu Gly Asn
            260                 265                 270
Val Ile Leu Phe Ile Asp Glu Leu His Thr Met Val Gly Ala Gly Lys
        275                 280                 285
Ala Asp Gly Ala Met Asp Ala Gly Asn Met Leu Lys Pro Ala Leu Ala

290                 295                 300
Arg Gly Glu Leu His Cys Val Gly Ala Thr Thr Leu Asp Glu Tyr Arg
305                 310                 315                 320
Gln Tyr Ile Glu Lys Asp Ala Ala Leu Glu Arg Arg Phe Gln Lys Val
                325                 330                 335
Phe Val Ala Glu Pro Ser Val Glu Asp Thr Ile Ala Ile Leu Arg Gly
            340                 345                 350
Leu Lys Glu Arg Tyr Glu Leu His His His Val Gln Ile Thr Asp Pro
        355                 360                 365
Ala Ile Val Ala Ala Ala Thr Leu Ser His Arg Tyr Ile Ala Asp Arg
    370                 375                 380
Gln Leu Pro Asp Lys Ala Ile Asp Leu Ile Asp Glu Ala Ala Ser Ser
385                 390                 395                 400
Ile Arg Met Gln Ile Asp Ser Lys Pro Glu Glu Leu Asp Arg Leu Asp
                405                 410                 415
Arg Arg Ile Ile Gln Leu Lys Leu Glu Gln Gln Ala Leu Met Lys Glu
            420                 425                 430
Ser Asp Glu Ala Ser Lys Lys Arg Leu Asp Met Leu Asn Glu Glu Leu
        435                 440                 445
Ser Asp Lys Glu Arg Gln Tyr Ser Glu Leu Glu Glu Glu Trp Lys Ala
    450                 455                 460
Glu Lys Ala Ser Leu Ser Gly Thr Gln Thr Ile Lys Ala Glu Leu Glu
465                 470                 475                 480
Gln Ala Lys Ile Ala Ile Glu Gln Ala Arg Arg Val Gly Asp Leu Ala
                485                 490                 495
Arg Met Ser Glu Leu Gln Tyr Gly Lys Ile Pro Glu Leu Glu Lys Gln
            500                 505                 510
Leu Glu Ala Ala Thr Gln Leu Glu Gly Lys Thr Met Arg Leu Leu Arg
        515                 520                 525
Asn Lys Val Thr Asp Ala Glu Ile Ala Glu Val Leu Ala Arg Trp Thr
    530                 535                 540
Gly Ile Pro Val Ser Arg Met Met Glu Ser Glu Arg Glu Lys Leu Leu
545                 550                 555                 560
Arg Met Glu Gln Glu Leu His His Arg Val Ile Gly Gln Asn Glu Ala
                565                 570                 575
Val Asp Ala Val Ser Asn Ala Ile Arg Arg Ser Arg Ala Gly Leu Ala
            580                 585                 590
Asp Pro Asn Arg Pro Ile Gly Ser Phe Leu Phe Leu Gly Pro Thr Gly
        595                 600                 605
Val Gly Lys Thr Glu Leu Cys Lys Ala Leu Ala Asn Phe Met Phe Asp
    610                 615                 620
Ser Asp Glu Ala Met Val Arg Ile Asp Met Ser Glu Phe Met Glu Lys
625                 630                 635                 640
His Ser Val Ser Arg Leu Val Gly Ala Pro Pro Gly Tyr Val Gly Tyr
                645                 650                 655
Glu Glu Gly Gly Tyr Leu Thr Glu Ala Val Arg Arg Arg Pro Tyr Ser
            660                 665                 670
Val Ile Leu Leu Asp Glu Val Glu Lys Ala His Pro Asp Val Phe Asn
        675                 680                 685
Ile Leu Leu Gln Val Leu Asp Asp Gly Arg Leu Thr Asp Gly Gln Gly
    690                 695                 700
Arg Thr Val Asp Phe Arg Asn Thr Val Val Ile Met Thr Ser Asn Leu
705                 710                 715                 720
Gly Ser Asp Leu Ile Gln Glu Arg Phe Gly Glu Leu Asp Tyr Ala His
                725                 730                 735
Met Lys Glu Leu Val Leu Gly Val Val Ser His Asn Phe Arg Pro Glu
            740                 745                 750
Phe Ile Asn Arg Ile Asp Glu Val Val Val Phe His Pro Leu Gly Glu
        755                 760                 765
Gln His Ile Ala Ser Ile Ala Gln Ile Gln Leu Lys Arg Leu Tyr Lys
    770                 775                 780
Arg Leu Glu Glu Arg Gly Tyr Glu Ile His Ile Ser Asp Glu Ala Leu
785                 790                 795                 800

Lys Leu Leu Ser Glu Asn Gly Tyr Asp Pro Val Tyr Gly Ala Arg Pro
                805                 810                 815
Leu Lys Arg Ala Ile Gln Gln Gln Ile Glu Asn Pro Leu Ala Gln Gln
            820                 825                 830
Ile Leu Ser Gly Glu Leu Val Pro Gly Lys Val Ile Arg Leu Glu Val
        835                 840                 845
Asn Glu Asp Arg Ile Val Ala Val Gln
    850                 855

## Description

### FIELD OF INVENTION

The present invention relates to the treatment or prevention of inflammation, such as obesity, overweight and/or obesity-related diseases and disorders. The present invention relates to methods of inducing satiation, prolonging satiety, reducing food intake, controlling weight gain and stimulating weight loss.

### BACKGROUND OF INVENTION

Obesity is one of such poorly treatable chronic conditions accompanied by numerous comorbidities. Obesity and overweight are typically characterized by increased food intake and decreased energy expenditure suggesting altered role of peptidergic systems regulating energy balance. Indeed, the regulation of appetite and feeding behavior involves interaction between intestinal hunger and satiety peptide hormones with the brain neuronal circuitries containing orexigenic and anorexigenic neuropeptides (Schwartz et al., 2000. Nature. 404(6778):661-71).

The current model of food intake control implicates gut-derived hunger and satiety hormones signalling to several brain circuitries regulating homeostatic and hedonic aspects of feeding (Berthoud H.-R., 2011. Curr. Opin. Neurobiol. 21(6):888-896; Murphy et al., 2006. Nature. 444(7121):854-859). Prominent amongst these are the anorexigenic and orexigenic pathways originating from the hypothalamic arcuate nucleus (ARC) that include the proopiomelanocortin (POMC) and neuropeptide Y (NPY)/agouti-related protein (AgRP) neurons, respectively, relayed in the paraventricular nucleus (PVN) (Atasoy et al., 2012. Nature. 488(7410):172-177; Garfield et al., 2015. Nat. Neurosci. 18(6):863-71; Shi et al., 2013. Cell Metab. 17(2):236-248).

The central melanocortin (MC) system consisting of melanocortin peptides including α-melanocyte-stimulating hormone (α-MSH) derived from its precursor proopiomelanocortin (POMC) and acting on the MC type 4 receptors (MC4R) is critically involved in regulation of energy balance (Cone, 2006. Endocr. Rev. 27(7):736-49). In fact, deficit in both POMC expression and MC4R signalling leads to hyperphagia and obesity in both human and genetically modified rodents (Huszar et al., 1997. Cell. 88(1):131-41; Krude et al., 1998. Nat. Genet. 19(2):155-7; Farooqi et al., 2003. N. Engl. J. Med. 348(12):1085-95). Selective stimulation of the central MC4R appears hence as a very attractive target to treat hyperphagia and obesity and several α-MSH peptide analogues have been developed and clinically tested, such as for instance setmelanotide as a replacement therapy during POMC deficiency and for treatment hyperphagia in Prader-Willi syndrome (Chen et al., 2015. J. Clin. Endocrinol. Metab. 100(4):1639-45; Kühnen et al., 2016. N. Engl. J. Med. 375(3):240-6). The penetration of high affinity α-MSH like drugs in the brain has however the risk of unwanted side-effects such as increasing blood pressure.

Recently, the gut commensal *Escherichia coli* (*E. coli*) caseinolytic protease B (ClpB) protein has been identified as a conformational mimetic of α-MSH, suggesting potential use of specific bacterial proteins as a new type of peptide-like drugs (Tennoune et al., 2014. Transl. Psychiatry. 4:e458). The ClpB protein has has a molecular weight superior to 95 kDa, which raises some difficulties (such as for example production, stability, and the like).

It was shown that activation of the MC4R in the gut enteroendocrine cells stimulates release of satiety hormones glucagon-like peptide-1 (GLP-1) and peptide YY (PYY) (Panaro et al., 2014. Cell Metab. 20(6):1018-1029).

A recent study showed that a ClpB fragment corresponding to amino acid residues 534-548 of ClpB sequence from *E. coli* is a full micromolar MC1R agonist with partial activities on MC3R and MC5R but not on MC4R (Ericson et al., Bioorganic & Medicinal Chemistry Letters, 2015, 25:5306-5308). However, the activity of the full ClpB or larger fragments containing α-MSH-like epitope on MCR may differ from the modified short fragment studied by Ericson *et al.*

The hormone α-MSH is also known to have potent anti-inflammatory effects and protective effects on cells of the immune system and on peripheral nonimmune cell types expressing melanocortin receptors, such as MC1R and MC3R (Brzoska et al., 2008. Endrocr. Rev. 29(5):581-602). Moreover, recent studies show that α-MSH is an interesting target for treating psoriasis, allergic rhinitis, osteoarthritis and neuroinflammatory diseases (Auriemma et al., 2012. J. Invest. Dermatol. 132(7):1814-24; Kleiner et al., Clin. Exp. Allergy. 46(8):1066-74; Böhm et al., 2016. Biochem. Pharmacol. 116:89-99; Mykicki et al., 2016. Sci. Transl. Med. 8(362):362ra146).

Therefore, there is still a need to identify new polypeptides or proteins that retain the biological activity of α-MSH, in particular binding to melanocortin receptors.

The inventors surprisingly showed that ClpB fragments comprising a sequence homology with α-MSH epitope are responsible of a direct action on intestinal mucosal cells to stimulate secretion of PYY.

The present invention thus relates to polypeptides and proteins comprising a fragment of a ClpB protein or variant thereof, and uses thereof.

### SUMMARY

The present invention relates to a polypeptide or protein of at least 15 amino acids comprising a fragment of a ClpB protein or variant thereof, comprising a negatively charged residue and two consecutive Arg and Trp residues, wherein said polypeptide or protein is not a full-length ClpB protein.

In one embodiment, the polypeptide or protein of the invention comprises the amino acid sequence SEQ ID NO: 2. In one embodiment, the polypeptide or protein of the invention comprises the amino acid sequence SEQ ID NO: 3. In one embodiment, the polypeptide or protein of the invention comprises the amino acid sequence SEQ ID NO: 4.

In one embodiment, the polypeptide or protein of the invention further comprises a sequence having at least 75% sequence homology with the sequence GKPV. In one embodiment, the polypeptide or protein of the invention comprises the amino acid sequence SEQ ID NO: 6.

The present invention also relates to a composition comprising the polypeptide or protein as described herein.

The present invention further relates to a pharmaceutical composition comprising the polypeptide or protein as described herein and at least one pharmaceutically acceptable excipient.

Another object of the present invention is a medicament comprising the polypeptide or protein according to the invention.

The present invention also relates to a dietary supplement comprising the polypeptide or protein as described herein. The present invention further relates to a food composition comprising the polypeptide or protein as described herein.

The present invention further relates to the polypeptide or protein, the pharmaceutical composition, or the medicament according to the invention for use in the treatment or prevention of obesity, overweight and/or obesity-related diseases and disorders in a subject in need thereof.

Another object of the present invention is the use of the polypeptide or protein, the dietary supplement or the food composition according to the invention for reducing weight in a subject. In one embodiment, the subject is not obese.

The present invention further relates to a kit comprising a polypeptide or protein, a composition, a pharmaceutical composition, a medicament, a dietary supplement or a food composition according to the invention.

### DEFINITIONS

In the present invention, the following terms have the following meanings:
- **"About"** preceding a figure means plus or less 10% of the value of said figure.
- **"Amino acids",** as used herein, are represented by their full name, their three letter code or their one letter code as well known in the art. Amino acid residues in peptides are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.
   The term **"amino acids"** includes both natural and synthetic amino acids, and both D and L amino acids. **"Standard amino acid"** or **"naturally occurring amino acid"** means any of the twenty standard L-amino acids commonly found in naturally occurring peptides. **"Non-standard amino acid residue"** means any amino acid, other than the standard amino acids, regardless of whether it is prepared synthetically or derived from a natural source. For example, naphtlylalanine can be substituted for tryptophan to facilitate synthesis. Other synthetic amino acids that can be substituted include, but are not limited to, L-hydroxypropyl, L-3,4-dihydroxyphenylalanyl, et-amino acids such as L-α-hydroxylysyl and D-α-methylalanyl, L-α-methylalanyl, β-amino acids, and isoquinolyl.
   The term **"amino acid"** also encompasses chemically modified amino acids, including but not limited to salts, amino acid derivatives (such as amides), and substitutions. Amino acids contained within the polypeptides of the present invention, and particularly at the carboxy- or amino-terminus, can be modified by methylation, amidation, acetylation or substitution with other chemical groups which can change the polypeptide's circulating half-life without adversely affecting their activity. Additionally, a disulphide linkage may be present or absent in the polypeptides of the invention.
- **"Antibody"** and **"immunoglobulin" ("Ig")** are interchangeable and include monoclonal antibodies, polyclonal antibodies, multispecific antibodies (e.g., bispecific antibodies), and antibody fragments, so long as they exhibit the desired biological activity.
- "**Binge eating", "compulsive eating",** "binge eating disorder" and **"compulsive eating disorder"** refer to an eating disorder consisting of episodes of uncontrollable eating, but without subsequent purging episodes (e.g., vomiting). **"Binge eaters"** are identified as experiencing binge- or compulsive eating based upon a Binge Eating Scale checklist (Gormally et al., 1982. Addict Behav. 7(1):47-55) or an equivalent diagnostic measure (e.g., professional assessment). Binge or compulsive eating severity is measured by the severity of individual events and/or by the frequency of such events.

- **"Conformational mimetic"** refers to a polypeptide or protein that shares at least in part the same conformation as another protein. In one embodiment, a "conformational mimetic of the α-MSH peptide" means a polypeptide or protein that shares at least in part the same conformation as the α-MSH peptide (SEQ ID NO: 20). In a particular embodiment, the conformational mimetic of the α-MSH peptide has consecutive Arg and Trp residues, a sequence having at least 75% sequence homology with the sequence GKPV, and/or a negatively charged residue upstream of the consecutive Arg and Trp.
- **"Conservative substitution"** is one in which an amino acid is substituted by another amino acid that has similar properties, such that one skilled in the art of peptide chemistry would expect the secondary structure and hydropathic nature of the polypeptide to be substantially unchanged. Amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine. Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include histidine, lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include:
   (1) Ala, Pro, Gly, Glu, Asp, Gln, Asn, Ser, Thr;
   (2) Cys, Ser, Tyr, Thr;
   (3) Val, Ile, Leu, Met, Ala, Phe;
   (4) Lys, Arg, His; and
   (5) Phe, Tyr, Trp, His.
   The term "**conservative amino acid substitution"** may further be defined as an amino acid exchange within one of the following five groups:
   (i) small aliphatic nonpolar or slightly polar residues: Ala, Ser, Thr, Pro, Gly;
   (ii) polar, negatively charged residues and their amides: Asp, Asn, Glu, Gln;
   (iii) polar, positively charged residues: His, Arg, Lys;
   (iv) large aliphatic nonpolar residues: Met, Leu, Ile, Val, Cys;
   (v) large aromatic residues: Phe, Tyr, Trp.
- **"Epitope"** refers to a specific arrangement of amino acids located on a protein or proteins to which an antibody binds. Epitopes often consist of a chemically active surface grouping of molecules such as amino acids or sugar side chains, and have specific three dimensional structural characteristics as well as specific charge characteristics. Epitopes can be linear and/or conformational, *i.e.,* involving two or more sequences of amino acids in various regions of the antigen that may not necessarily be contiguous.
- **"Fragment"** refers to a part or a region of a protein, e.g., of the ClpB protein, comprising fewer amino acid residues than an intact or complete protein, e.g., the ClpB protein. The term **"fragment"** further refers to, for example, an at least about 5, 10, 20, 30, 40, 50, 75, 100, 150, 200, 250, 300, 400, 500, 600, 700, 800 or more amino acid portion of an amino acid sequence, e.g., of amino acid sequence SEQ ID NO: 1, which portion is cleaved from a naturally occurring amino acid sequence by proteolytic cleavage by at least one protease, or is a portion of the naturally occurring amino acid sequence synthesized by chemical methods or using recombinant DNA technology (e.g., expressed from a portion of the nucleotide sequence encoding the naturally occurring amino acid sequence) known to one of skill in the art. **"Fragment"** may also refer to a portion, for example, of about 5%, about 10%, about 20%, about 30%, about 40%, about 50%, about 60%, about 70%, about 80% about 90% about 95% or about 99% of a particular amino acid sequence, e.g., of amino acid sequence SEQ ID NO: 1.
- **"Identity"** or **"identical",** when used in a relationship between the sequences of two or more amino acid sequences, refers to the degree of sequence relatedness between amino acid sequences, as determined by the number of matches between strings of two or more amino acid residues. **"Identity"** measures the percent of identical matches between the smaller of two or more sequences with gap alignments (if any) addressed by a particular mathematical model or computer program (*i.e.,* **"algorithms"**). Methods for comparing the identity of two or more sequences are well known in the art. Such methods include, but are not limited to, those described in Arthur M. Lesk, Computational Molecular Biology: Sources and Methods for Sequence Analysis (New-York: Oxford University Press, 1988); Douglas W. Smith, Biocomputing: Informatics and Genome Projects (New-York: Academic Press, 1993); Hugh G. Griffin and Annette M. Crriffin, Computer Analysis of Sequence Data, Part 1 (New Jersey: Humana Press, 1994); Gunnar von Heinje, Sequence Analysis in Molecular Biology: Treasure Trove or Trivial Pursuit (Academic Press, 1987); Michael Gribskov and John Devereux, Sequence Analysis Primer (New York: M. Stockton Press, 1991); and Carillo et al., 1988. SIAM J. Appl. Math. 48(5):1073-1082. Preferred methods for determining identity are designed to give the largest match between the sequences tested. Methods of determining identity are described in publicly available computer programs. Computer program methods for determining identity between two sequences include the GCG program package, including GAP (Devereux et al., 1984. Nucl. Acid. Res. 12(1 Pt 1):387-395; Genetics Computer Group, University of Wisconsin Biotechnology Center, Madison, WI), BLASTP, BLASTN, TBLASTN and FASTA (Altschul et al., 1990. J. Mol. Biol. 215(3): 403-410). The BLASTX program is publicly available from the National Center for Biotechnology Information (NCBI) and other sources (BLAST Manual, Altschul et al., NCB/NLM/NIH Bethesda, Md. 20894; Altschul et al, 1990. J. Mol. Biol. 215(3):403-410). The "needle" program, which uses the Needleman-Wunsch global alignment algorithm (Needleman S.B. and Wunsch C.D., 1970. J. Mol. Biol. 48:443-453) to find the optimum alignment (including gaps) of two sequences when considering their entire length, may preferably be used. The needle program is, for example, available on the ebi.ac.uk world wide web site. The percentage of identity in accordance with the invention is preferably calculated using the EMBOSS: needle (global) program with a "Gap Open" parameter equal to 10.0, a "Gap Extend" parameter equal to 0.5, and a Blosum62 matrix. The well-known Smith Waterman algorithm may also be used to determine identity.
- **"Obesity"** refers to a medical condition wherein the subject preferably has a BMI of > 30. The **"BMI"** or **"body mass index"** is defined as the subject's body mass divided by the square of his height. The formulae universally used in medicine produces a unit of measure of kg/m². A **"moderately obese"** subject refers to a subject having a BMI of between 30 and 35. A **"non-obese"** subject has a normal body weight. **"Normal body weight"** refers herein to body weight resulting in a BMI of between 18.5 and 25.
- **"Overweight"** refers to body weight resulting in a BMI of between 25 and 30. In some embodiments, the subject is a healthy overweight or uncomplicated overweight subject. By **"healthy overweight"** or **"uncomplicated overweight"** subject is meant herein an overweight subject who does not display any disease or condition directly associated with his/her weight.
- **"Pharmaceutically"** or **"pharmaceutically acceptable"** refer to molecular entities and compositions that do not produce an adverse, allergic or other untoward reaction when administered to a subject, especially a human, as appropriate. A pharmaceutically acceptable carrier or excipient refers to a non-toxic solid, semi-solid or liquid filler, diluent, encapsulating material or formulation auxiliary of any type. Pharmaceutically acceptable excipients that may be used in the compositions of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The active ingredient can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatine. Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion. Some variation in dosage will necessarily occur depending on the condition of the subject being treated. The person responsible for administration will, in any event, determine the appropriate dose for the individual subject.

- **"Polypeptide"** is used in its conventional meaning, *i.e.,* as a sequence of less than 100 amino acids. A polypeptide usually refers to a monomeric entity. The term **"protein"** refers to a sequence of more than 100 amino acids and/or to a multimeric entity. The proteins of the invention are not limited to a specific length of the product. This term does not refer to or exclude post-expression modifications of the protein, for example, glycosylation, acetylation, phosphorylation and the like, as well as other modifications known in the art, both naturally occurring and non-naturally occurring. A protein may be an entire protein, or a subsequence thereof. An **"isolated protein"** is one that has been identified and separated and/or recovered from a component of its natural environment. In preferred embodiments, the isolated protein will be purified:
   (1) to greater than 80, 85, 90, 95% by weight of protein as determined by the Lowry method, and most preferably more than 96, 97, 98, or 99% by weight;
   (2) to a degree sufficient to obtain at least 15 residues of N-terminal or internal amino acid sequence by use of a spinning cup sequenator; or
   (3) to homogeneity by SDS-PAGE under reducing or non-reducing conditions using Coomassie blue or, preferably, silver staining.
   Isolated protein includes the protein *in situ* within recombinant cells since at least one component of the protein's natural environment will not be present. Ordinarily, however, isolated protein will be prepared by at least one purification step.
- **"Polypeptide derivative"** or **"protein derivative"** refers to compound having an amino group (--NH--), and more particularly, a peptide bond. Polypeptides and proteins may be regarded as substituted amides. Like the amide group, the peptide bond shows a high degree of resonance stabilization. The C--N single bond in the peptide linkage has typically about 40 percent double-bond character and the C=O double bond about 40 percent single-bond character. **"Protecting groups"** are those groups that prevent undesirable reactions (such as proteolysis) involving unprotected functional groups. Specific examples of amino protecting groups include formyl; trifluoroacetyl; benzyloxycarbonyl; substituted benzyloxycarbonyl such as (ortho- or para-) chlorobenzyloxycarbonyl and (ortho- or para-) bromobenzyloxycarbonyl; and aliphatic oxycarbonyl such as t-butoxycarbonyl and t-amiloxycarbonyl. The carboxyl groups of amino acids can be protected through conversion into ester groups. The ester groups include benzyl esters, substituted benzyl esters such as methoxybenzyl ester; alkyl esters such as cyclohexyl ester, cycloheptyl ester or t-butyl ester. The guanidino moiety may be protected by nitro; or arylsulfonyl such as tosyl, methoxybenzensulfonyl or mesitylenesulfonyl, even though it does not need a protecting group. The protecting groups of imidazole include tosyl, benzyl and dinitrophenyl. The indole group of tryptophan may be protected by formyl or may not be protected.
   The modification of the polypeptide or protein comprising or consisting of at least a fragment of a ClpB protein or variant thereof aims, in particular, to improve their life time *in vivo.* One type of modification is the addition to the N or C termini of the polypeptide or protein of polyethylene glycol (PEG). PEG is known by the person skilled in the art to have many properties that make it an ideal carrier for polypeptides such as high water solubility, high mobility in solution and low immunogenicity. This modification also protects the polypeptides and proteins from exopeptidases and therefore increases their overall stability *in vivo.*
   The other modifications used to prevent degradation of polypeptides and proteins by endopeptidases or exopeptidases include N-terminal modifications such as acetylation or glycosylation, C-terminal modifications such as amidation and use of unnatural amino acids (β-amino and α-trifluoromethyl amino acids) at particular sites within the polypeptides.
   Another alternative to increase polypeptides and proteins molecular size is the genetic fusion of the polypeptides to the Fc domain of human immunoglobulin (including, for example, IgA, IgM and IgG) or the fusion of the polypeptides to albumin.
- **"Satiety"** refers to an essentially homeostatic state wherein an individual feels that their cravings are satisfied or minimized. Many physiological factors are believed to bear on an individual's satiety. For instance, gustation, or taste, olfaction, or smell, as well as a feeling of fullness of the stomach may all contribute to whether an individual feels **"satiated."** More in particular, **"satiety"** is the state in which further eating is inhibited and determines the time between meals and the amount of food consumed at the next meal. An **"enhanced feeling of satiety"** or the like, this has the meaning of the feeling of satiety being more pronounced and/or more prolonged compared to a control situation.
- "**Satiation"** refers to the state which terminates eating within a meal, typically occurring/observed within a period (e.g. 20-30 min) after the start of consuming the meal. Thus, whenever reference is made in this document to "inducing satiation" or the like, this has the meaning of arousing the tendency of a subject to stop consuming food during a meal. The effect on satiation can be determined by scoring the time point of meal termination. A satiation effect is seen if the amount of consumed calories at meal termination is significantly less than in the controls, such as for example at least 1%, 2%, 3%, 4%, 5%, 10% 20%, or more. Over a longer time period (such as 1, 2, 3, 4, 5 weeks or more), one can also score the body weight reduction or the body weight change compared to a control diet. Body weight of a subject being administered regular amounts of the test compositions (e.g. once daily, twice daily, or more) is preferably significantly controlled (reduced or less increased) compared to the control subjects. As used herein, the **"control subject"** refers to the subjects who were not administered with the polypeptide or protein, polynucleotide, vector, composition, pharmaceutical composition, medicament or vaccine of the invention.
- **"Subject"** refers to a warm-blooded animal, preferably a human, a pet or livestock. As used herein, the terms **"pet"** and **"livestock"** include, but are not limited to, dogs, cats, guinea pigs, rabbits, pigs, cattle, sheep, goats, horses and poultry. In some embodiments, the subject is a male or female subject. In some embodiments, the subject is an adult (for example, a subject above the age of 18 [in human years] or a subject after reproductive capacity has been attained). In another embodiment, the subject is a child (for example, a subject below the age of 18 [in human years] or a subject before reproductive capacity has been attained). In some embodiments, the subject may be a **"patient",** *i.e.,* a subject who/which is awaiting the receipt of, or is receiving medical care or was/is/will be the object of a medical procedure according to the methods of the present invention, or is monitored for the development of a disease.

- **"Sustained release"** indicates that the therapeutically active agent may be released from the composition at a controlled rate in such a manner that blood levels (that are still below the toxic levels of the medicament) may be maintained at therapeutically beneficial levels over an extended duration of time (e.g., 24 hours or more, thereby providing a single dose, daily dosage formulation).
- **"Therapeutically effective amount"** refers to a quantity of:
   o polypeptide or protein comprising or consisting of at least a fragment of the ClpB protein or variant thereof; or
   o polynucleotide encoding a polypeptide or protein comprising or consisting of at least a fragment of the ClpB protein or variant thereof as described herein; or
   o vector encoding a polypeptide or protein comprising or consisting of at least a fragment of the ClpB protein or variant thereof as described herein;
   sufficient to, without causing significant negative or adverse side effects to the subject, achieve the beneficial effect (e.g. stimulating satiety, prolonging satiation, reducing food intake, controlling, in particular reducing, weight gain, stimulating weight loss, and/or reducing fat mass on lean mass ratio). In the context of the present invention, the amount of polypeptide, protein, polynucleotide or vector to be administered to the subject will depend on the characteristics of the individual, such as general health, age, sex, body weight... The skilled artisan will be able to determine appropriate dosages depending on these and other factors.
- **"Treating", "treatment"** or **"alleviation"** refer to both therapeutic treatment and prophylactic or preventative measures; wherein the object is to prevent or slow down (lessen) the targeted condition or disorder. Those in need of treatment include those already with the disorder as well as those prone to have the disorder or those in whom the disorder is to be prevented. A subject or mammal is successfully **"treated"** for the targeted condition or disorder if, after receiving a therapeutic amount of an agent according to the present invention, the subject shows observable and/or measurable: satiation, prolonged satiety, reduced food intake, controlled weight gain, stimulated weight loss and/or reduced fat mass on lean mass ratio. These parameters for assessing successful treatment and improvement in the condition or disorder are readily measurable by routine procedures familiar to a physician.
- **"Variant"** and **"mutant"** are interchangeable terms, and refer to a polypeptide that typically differs from a polypeptide specifically disclosed herein in one or more substitutions, deletions, additions and/or insertions. Such variants may be naturally occurring or may be synthetically generated, for example, by modifying a polypeptide sequence and evaluating one or more biological activities of the polypeptide as described herein and/or using any of a number of techniques well known in the art. Modifications may be made in the structure of polypeptides and still obtain a functional molecule that encodes a variant or derivative polypeptide with desirable characteristics.
   When it is desired to alter the amino acid sequence of a polypeptide to create an equivalent, or even an improved, variant or portion of a ligand of the invention, one skilled in the art will typically change one or more of the codons of the encoding DNA sequence. For example, certain amino acids may be substituted by other amino acids in a protein structure without appreciable function loss as compared to the wild-type protein, e.g., to the ClpB protein.
   Certain amino acid sequence substitutions, deletions, additions and/or insertions can be made in a protein sequence, and, of course, its underlying DNA coding sequence, and therefore obtain a protein with different properties. It is thus contemplated that various changes may be made in the peptide sequences, or corresponding DNA sequences that encode said peptides with appreciable regulation (*i.e.,* activation/enhancement or inhibition/loss) of their biological utility or activity. Such mutations include but are not limited to, non-sense mutations, frameshift mutations, and non-conservative missense mutations.
   Certain amino acid sequence substitutions, deletions, additions and/or insertions can be made in a protein sequence, and, of course, its underlying DNA coding sequence, and nevertheless obtain a protein with similar properties. It is thus contemplated that various changes may be made in the peptide sequences, or corresponding DNA sequences that encode said peptides without appreciable loss of their biological utility or activity. Such mutations include but are not limited to, silent mutations, silent missense mutations and conservative mutations.
   The terms **"variant"** and **"mutant"** also encompass polypeptide having one or more of the following modifications:
   ii) polypeptides wherein one or more of the peptidyl -C(O)NR- linkages (bonds) have been replaced by a non-peptidyl linkage such as a -CH₂-carbamate linkage (-CH₂OC(O)NR-), a phosphonate linkage, a -CH₂-sulfonamide (-CH₂-S(O)₂NR-) linkage, a urea (-NHC(O)NH-) linkage, a -CH₂-secondary amine linkage, or with an alkylated peptidyl linkage (-C(O)NR-) wherein R is C₁-C₄ alkyl;
   iii) polypeptides wherein the N-terminus is derivatized to a -NRR₁ group, to a -NRC(O)R group, to a -NRC(O)OR group, to a -NRS(O)₂R group, to a -NHC(O)NHR group where R and R₁ are hydrogen or C₁-C₄ alkyl with the proviso that R and R₁ are not both hydrogen;
   iv) polypeptides wherein the C terminus is derivatized to -C(O)R₂ where R₂ is selected from the group consisting of C₁-C₄ alkoxy, and -NR₃R₄ where R₃ and R₄ are independently selected from the group consisting of hydrogen and C₁-C₄ alkyl.
- **"Wild-type"** refers to a gene or a protein that has the characteristics of that gene or protein isolated from a naturally occurring source. A wild-type gene or protein is that which is most frequently observed in a population and is thus arbitrarily designated the **"wild-type"** form of the gene or protein, by contrast to a **"variant"** or **"mutant".**

### DETAILED DESCRIPTION

The present invention thus relates to a polypeptide or protein comprising or consisting of at least a fragment of a ClpB protein or variant thereof.

In one embodiment, the polypeptide or protein of the invention comprises or consists of at least 15 amino acids. In one embodiment, the polypeptide or protein of the invention comprises or consists of at least 16, 17, 18, 19, 20, 25, 30, 35, 40, 50, 60, 75, 100 or more amino acids. In one embodiment, the polypeptide of the invention comprises or consists of less than 100, 75, 60, 50, 40, 35, 30, 25, 20, 19, 18, 17, or 16 amino acids in length.

In one embodiment, the polypeptide of the invention comprises from 4 to 100 or more amino acids, from 10 to 80 amino acids, from 15 to 70 amino acids, from 20 to 60 amino acids, from 25 to 50 amino acids, from 30 to 45 amino acids, or from 35 to 40 amino acids.

In another embodiment, the polypeptide of the invention comprises from 10 to 100 amino acids, or from 10 to 80, 70, 60, 50, 45, 40, 35, 30, 25, 20 or 15 amino acids. In another embodiment, the polypeptide of the invention comprises from 15 to 100 amino acids, or from 15 to 80, 70, 60, 50, 45, 40, 35, 30, 25, or 20 amino acids. In another embodiment, the polypeptide of the invention comprises from 20 to 100 amino acids, or from 20 to 80, 70, 60, 50, 45, 40, 35, 30, or 25 amino acids. In another embodiment, the polypeptide of the invention comprises from 25 to 100 amino acids, or from 25 to 80, 70, 60, 50, 45, 40, 35, or 30 amino acids.

In one embodiment, the polypeptide of the invention comprises or consists of 15, 16, 17, 18, 19, 20, 21, 22, 23 or 24 amino acids. In another embodiment, the polypeptide of the invention comprises or consists of 25, 30, 35, 40, 45, 50, 55, 60, 65, 70, 75, 80, 85, 90, 95 or 100 amino acids.

In one embodiment, the polypeptide of the invention does not comprise less than 15 amino acids. In one embodiment, the polypeptide of the invention comprises at least 15 amino acids. In one embodiment, the polypeptide of the invention does not comprise less than 20 amino acids. In one embodiment, the polypeptide of the invention comprises at least 20 amino acids.

In another embodiment, the protein of the invention comprises or consists of at least 100, 125, 150, 200, 250, 300, 350, 400, 450, 500, 550, 600 or more amino acids.

In one embodiment, the protein of the invention comprises from 100 to 1500 or more amino acids, from 100 to 1000 amino acids, from 100 to 900 amino acids, from 100 to 850 amino acids, from 100 to 800 amino acids, from 100 to 750 amino acids, or from 100 to 700 amino acids. In another embodiment, the protein of the invention comprises from 150 to 1500, 1000, 900, 850, 800, 750 or 700 amino acids. In another embodiment, the protein of the invention comprises from 200 to 1500, 1000, 900, 850, 800, 750 or 700 amino acids. In another embodiment, the protein of the invention comprises from 300 to 1500, 1000, 900, 850, 800, 750 or 700 amino acids.

In one embodiment, the polypeptide or protein of the invention comprises from 15 to 1500 or more amino acids, from 15 to 1000 amino acids, from 15 to 900 amino acids, from 15 to 800 amino acids, from 15 to 700 amino acids, from 15 to 600 amino acids, or from 15 to 500 amino acids. In another embodiment, the polypeptide or protein of the invention comprises from 20 to 1500, 1000, 900, 800, 700, 600 or 500 amino acids. In another embodiment, the polypeptide or protein of the invention comprises from 25 to 1500, 1000, 900, 800, 700, 600 or 500 amino acids. In another embodiment, the polypeptide or protein of the invention comprises from 30 to 1500, 1000, 900, 800, 700, 600 or 500 amino acids.

A ClpB protein according to the present invention refers to a caseinolytic protease B protein, which is a member of the Hsp100/ClpB family of hexameric AAA+-ATPases.

In one embodiment, the ClpB protein according to the present invention is a bacterial ClpB protein. In one embodiment, the ClpB protein according to the present invention is a *Enterobacteriaceae* protein. *Enterobacteriaceae* include, but are not limited to, *Arsenophonus, Brenneria, Buchnera, Budvicia, Buttiauxella, Cedecea, Citrobacter, Cosenzaea, Cronobacter, Dickeya, Edwardsiella, Enterobacillus, Enterobacter, Erwinia, Escherichia, Ewingella, Franconibacter, Gibbsiella, Hafnia, Izhakiella, Kosakonia, Klebsiella, Kluyvera, Leclercia, Lelliottia, Leminorella, Levinea, Lonsdalea, Mangrovibacter, Moellerella, Morganella, Obesumbacterium, Pantoea, Pectobacterium, Phaseolibacter, Photorhabdus, Plesiomonas, Pluralibacter, Pragia, Proteus, Providencia, Pseudocitrobacter, Rahnella, Raoultella, Rosenbergiella, Rouxiella, Saccharobacter, Salmonella, Samsonia, Serratia, Shigella, Shimwellia, Siccibacter, Sodalis, Tatumella, Thorsellia, Trabulsiella, Wigglesworthia, Xenorhabdus, Yersinia* and *Yokenella.*

In one embodiment, the ClpB protein according to the present invention is a ClpB from *Hafnia,* preferably from *Hafnia alvei* (SEQ ID NO: 1). In one embodiment, the ClpB protein according to the present invention is a ClpB from *E. Coli,* preferably from *E. Coli* K12 (SEQ ID NO: 21).

In *Hafnia alvei,* the chaperone protein ClpB is a protein of 857 amino acids. In one embodiment, the ClpB protein has the amino acid sequence of the chaperone protein ClpB from *Hafnia alvei* with SEQ ID NO: 1 (GenBank Reference Number: KIC99545.2). In one embodiment, the amino acid sequence of ClpB comprises or consists of an amino acid sequence at least about 95 to about 100% identical to the amino acid sequence of SEQ ID NO: 1. In the context of the present application, the percentage of identity is calculated using a global alignment (*i.e.* the two sequences are compared over their entire length).
**SEQ ID NO: 1** 
MRLDRLTSKFQLALADAQSLALGRDNQFIEPVHLMSALLNQDGGTVRPLLTSA
GVNTASLRQELEQAISRLPQVEGAGGDVQPSNELIRVLNLCDKLAQKRNDTFIS
SELFVLAVLEDRGNLGDILKAAGANVQKVSTAIEQMRGGEKVDDANAEDQRQ
ALKKYTVDLTERAEQGKLDPVIGRDEEIRRTIQVLQRRTKNNPVLIGEPGVGKT
AIVEGLAQRIINGEVPEGLKNKRVLSLDMGSLIAGAKFRGEFEERLKAVLSDLSK
QEGNVILFIDELHTMVGAGKGDGAMDAGNMLKPALARGELHCVGATTLDEYR
QYIEKDAALERRFQKVFVAEPTVEDTIAILRGLKERYELHHHVQITDPAIVAAA
NLSHRYIADRQLPDKAIDLIDEAASSIRMQMDSKPESLDRLERRIIQLKLEQQAL
NKESDEASKKRLEMLNEELEHKEREYSELEEEWKAEKASLSGTQNIKAEIEQAK
 
IALEQARRVGDLAKMSEIQYGKLPGLEKQLEAATQSEGKTMKLLRNKVTDVEI
AEVLARWTGIPVSRMLESERAKLLRMEDDLHERVIGQNEAVEAVSNAIRRSRA
GLSDPNRPIGSFLFLGPTGVGKTELCKALANFLFDSDDAMVRIDMSEFMEKHSV
SRLVGAPPGYVGYEEGGYLTEAVRRRPYSVILLDEVEKAHPDVFNILLQVLDDG
RLTDGQGRTVDFRNTVVIMTSNLGSDLIQDRFGQLDYGEMKELVMSVVSHHFR
PEFINRIDETVVFHPLDQKNIKAIARIQLERLYQRLEEKGYSVTITDAALEQLSKA
GFDPVYGARPLKRAIQQEIENPLAQQILSGKLIPGKDITLDVADEQIVAKQ
 In one embodiment, the ClpB protein according to the present invention is a ClpB variant.
   In one embodiment, a ClpB variant as used herein comprises or consists of a sequence presenting a sequence identity of at least about 70% with amino acid sequence SEQ ID NO: 1, preferably of at least about 80%, more preferably of at least about 90%, 91%, 92%, 93%, 94%, 95%, 96%, 97%, 98%, 99% or more.
   A ClpB variant may also, or alternatively, contain non-conservative changes. In one embodiment, variant polypeptides differ from a native sequence by substitution, deletion or addition of 1, 2, 3, 4, 5, 6, 7, 8, 9, 10 or more amino acids. Variants may also (or alternatively) be modified by, for example, the deletion or addition of amino acids that have minimal influence on the immunogenicity, secondary structure and hydropathic nature of the polypeptide.
   In one embodiment, a variant of SEQ ID NO: 1 is capable of inducing satiation, prolonging satiety, reducing food intake, controlling weight gain, stimulating weight loss and/or reducing fat mass on lean mass ratio in a subject in need thereof with an efficiency at least equivalent to the one of SEQ ID NO: 1.
   In one embodiment, a variant of SEQ ID NO: 1 comprises conservative amino acid substitutions as compared to the sequence of SEQ ID NO: 1, such as, for example, 1, 2, 3, 4, 5, 6, 7, 8, 9, 10 or more conservative amino acid substitutions.
   In another embodiment, a variant of SEQ ID NO: 1 is a polypeptide wherein 1, 2, 3, 4, 5, 6, 7, 8, 9, 10 or more amino acids from the sequence of SEQ ID NO: 1 is/are absent, or substituted by any amino acid, or wherein 1, 2, 3, 4, 5, 6, 7, 8, 9, 10 or more amino acids (either contiguous or not) is/are added.
   In one embodiment, the polypeptide or protein of the invention comprises or consists of at least a fragment of ClpB, preferably at least a fragment of the ClpB having the amino acid sequence SEQ ID NO: 1.
   In one embodiment, a ClpB fragment comprises or consists of at least 4, 5, 6, 7, 8, 9, 10, 11, 12, 13, 14, 15, 16, 17, 18, 19, 20, 25, 30, 35, 40, 50, 60, 75, 100 or more amino acids of a ClpB protein, preferably of a ClpB having the amino acid sequence SEQ ID NO:1.
   In one embodiment, a ClpB fragment comprises or consists of less than 100, 75, 60, 50, 40, 35, 30, 25, 20, 19, 18, 17, 16, 15, 14, 13, 12, 11, 10 or less amino acids in length.
   In one embodiment, a ClpB fragment comprises from 4 to 100 or more amino acids, from 10 to 80 amino acids, from 15 to 70 amino acids, from 20 to 60 amino acids, from 25 to 50 amino acids, from 30 to 45 amino acids, or from 35 to 40 amino acids.
   In another embodiment, a ClpB fragment comprises from 4 to 80 amino acids, or from 4 to 70, 60, 50, 45, 40, 35, 30, 25, 20, 15 or 10 amino acids. In another embodiment, a ClpB fragment comprises from 6 to 100 amino acids, or from 6 to 80, 70, 60, 50, 45, 40, 35, 30, 25, 20, 15 or 10 amino acids. In another embodiment, a ClpB fragment comprises from 8 to 100 amino acids, or from 8 to 80, 70, 60, 50, 45, 40, 35, 30, 25, 20, 15 or 10 amino acids. In another embodiment, a ClpB fragment comprises from 10 to 100 amino acids, or from 10 to 80, 70, 60, 50, 45, 40, 35, 30, 25, 20 or 15 amino acids.
   In one embodiment, a ClpB fragment comprises or consists of 4, 5, 6, 7, 8, 9, 10, 11, 12, 13, 14, 15, 16, 17, 18, 19 or 20 amino acids. In another embodiment, a ClpB fragment comprises or consists of 25, 30, 35, 40, 45, 50, 55, 60, 65, 70, 80, 90 or 100 amino acids.
   In another embodiment, a ClpB fragment comprises from 100 to 500 or more amino acids, from 100 to 400 amino acids, from 100 to 300 amino acids, from 100 to 200 amino acids, or from 100 to 150 amino acids. In another embodiment, a ClpB fragment comprises from 150 to 500 or more amino acids, from 150 to 400 amino acids, from 150 to 300 amino acids, or from 150 to 200 amino acids.
   In one embodiment, a ClpB fragment comprises or consists of 100, 125, 150, 175, 200, 250, or 300 amino acids.
   In one embodiment, a ClpB fragment comprises or consists of at least 4, 5, 6, 7, 8, 9, 10, 11, 12, 13, 14, 15, 16, 17, 18, 19, 20, 25, 30, 35, 40, 50, 60, 75, 100 or more amino acid residues of SEQ ID NO: 1 or variant thereof.
   In one embodiment, a ClpB fragment comprises or consists of at least 4, 5, 6, 7, 8, 9, 10, 11, 12, 13, 14, 15, 16, 17, 18, 19, 20, 25, 30, 35, 40, 50, 60, 75, 100 or more contiguous amino acid residues of SEQ ID NO: 1. In one embodiment, a ClpB fragment comprises or consists of at least 4, 5, 6, 7, 8, 9, 10, 11, 12, 13, 14, 15, 16, 17, 18, 19, 20, 25, 30, 35, 40, 50, 60, 75, 100 ormore discontiguous amino acid residues of SEQ ID NO: 1 or variant thereof. In one embodiment, a ClpB fragment comprises or consists of at least 5 discontiguous amino acid residues of SEQ ID NO: 1 or variant thereof. In one embodiment, a ClpB fragment comprises or consists of at least 6 discontiguous amino acid residues of SEQ ID NO: 1 or variant thereof.
   In one embodiment, a ClpB fragment of the invention comprises a negatively charged residue and two consecutive Arg and Trp residues. In one embodiment, a ClpB fragment of the invention comprises consecutive Arg and Trp residues and a negatively charged residue upstream of the consecutive Arg and Trp residues.
   In one embodiment, a ClpB fragment of the invention comprises at least a negatively charged residue upstream of consecutive Arg and Trp residues. As used herein, the term "upstream of consecutive Arg and Trp residues" means at position -1, -2, -3, -4 or -5 of the Arg residue. In others words, in one embodiment, the Arg residue of the consecutive Arg and Trp sequence is at position +1, +2, +3, +4 or +5 of the negatively charged residue.
   In one embodiment, the negatively charged residue is a Glu or Asp residue. In a particular embodiment, the negatively charged residue is a Glu residue.
   In one embodiment, a ClpB fragment comprises at least amino acid residue E538 of SEQ ID NO: 1 or variant thereof.
   In one embodiment, a ClpB fragment comprises or consists of at least amino acid residues R542 and/or W543 of SEQ ID NO: 1 or variant thereof. In one embodiment, a ClpB fragment comprises or consists of at least amino acid residues R542 and W543 of SEQ ID NO: 1 or variant thereof.
   In one embodiment, a ClpB fragment comprises or consists of at least amino acid residue E538, R542 and W543 of SEQ ID NO: 1.
   In one embodiment, a ClpB fragment comprises or consists of amino acid sequence SEQ ID NO: 2.
**SEQ ID NO: 2**

   E/D-X₂₋₄-R-W

   wherein X is any amino acid and X₂₋₄ is 2, 3 or 4 of any amino acid, preferably wherein E/D is E.
   In another particular embodiment, a ClpB fragment comprises or consists of amino acid sequence SEQ ID NO: 3.
**SEQ ID NO: 3**

   E/D-V-X-X-R-W

   wherein X is any amino acid, preferably wherein E/D is E.
   In another particular embodiment, a ClpB fragment comprises or consists of amino acid sequence SEQ ID NO: 4.
**SEQ ID NO: 4**

   E/D-V-L-A-R-W

   In a preferred embodiment, a ClpB fragment comprises or consists of amino acid sequence SEQ ID NO: 5.
**SEQ ID NO: 5**

   E-V-L-A-R-W

   In a particular embodiment, the polypeptide or protein of the invention comprises the amino acid sequence SEQ ID NO: 2, 3, 4 or 5 and has a length of at least 15 amino acids, preferably at least 20 amino acids.
   In one embodiment, a ClpB fragment of the invention further comprises a sequence having at least 75% sequence homology with the sequence GKPV.
   In one embodiment, a ClpB fragment further comprises at least amino acid residues G545, P547 and/or V548 of SEQ ID NO: 1 or variant thereof. In a particular embodiment, a ClpB fragment comprises at least amino acid residues G545,1546, P547 and/or V548 of SEQ ID NO: 1 or variant thereof. In a preferred embodiment, a ClpB fragment comprises at least amino acid residues G545, I546, P547 and V548 of SEQ ID NO: 1 or variant thereof.
   In one embodiment, a ClpB fragment according to the invention comprises or consists of at least amino acid residues E538, R542, W543, G545, P547 and V548 of SEQ ID NO: 1 or variant thereof. In one embodiment, a ClpB fragment according to the invention comprises or consists of at least amino acid residues E538, R542, W543, G545, I546, P547 and V548 of SEQ ID NO: 1 or variant thereof.
   In one embodiment, a ClpB fragment comprises or consists of amino acid sequence SEQ ID NO: 6.
**SEQ ID NO: 6**

   E/D-X₂₋₄-R-W-X-G-X-P-V

   wherein X is any amino acid and X₂₋₄ is 2, 3 or 4 of any amino acid, preferably wherein E/D is E.
   In one embodiment, a ClpB fragment comprises or consists of amino acid sequence SEQ ID NO: 7.
**SEQ ID NO: 7**

   E/D-X₂₋₄-R-W-X-G-I/K-P-V

   wherein X is any amino acid and X₂₋₄ is 2, 3 or 4 of any amino acid.
   In one embodiment, a ClpB fragment comprises or consists of amino acid sequence SEQ ID NO: 8.
**SEQ ID NO: 8**

   E-X₂₋₄-R-W-X-G-I/K-P-V

   In one embodiment, a ClpB fragment comprises or consists of amino acid sequence SEQ ID NO: 9.
**SEQ ID NO: 9**

   E-X₂₋₄-R-W-X-G-I-P-V

   wherein X is any amino acid and X₂₋₄ is 2, 3 or 4 of any amino acid.
   In one embodiment, a ClpB fragment comprises or consists of amino acid sequence SEQ ID NO: 10.
**SEQ ID NO: 10**

   E-X₂₋₄-R-W-T-G-I-P-V

   wherein X₂₋₄ is 2, 3 or 4 of any amino acid.
   In one embodiment, a ClpB fragment comprises or consists of amino acid sequence SEQ ID NO: 11.
**SEQ ID NO: 11**

   E-V-X-X-R-W-T-G-I-P-V

   wherein X is any amino acid.
   In one embodiment, a ClpB fragment comprises or consists of amino acid sequence SEQ ID NO: 12.
**SEQ ID NO: 12**

   E-V-L-A-R-W-T-G-I-P-V

   In one embodiment, a ClpB fragment comprises or consists of amino acid sequence SEQ ID NO: 13.
**SEQ ID NO: 13**

   E-X₂₋₄-R-W-X-G-K-P-V

   wherein X is any amino acid and X₂₋₄ is 2, 3 or 4 of any amino acid.
   In one embodiment, a ClpB fragment comprises or consists of amino acid sequence SEQ ID NO: 14.
**SEQ ID NO: 14**

   E-X₂₋₄-R-W-T-G-K-P-V

   wherein X₂₋₄ is 2, 3 or 4 of any amino acid.
   In one embodiment, a ClpB fragment comprises or consists of amino acid sequence SEQ ID NO: 15.
**SEQ ID NO: 15**

   E-V-X-X-R-W-T-G-K-P-V

   wherein X is any amino acid.
   In one embodiment, a ClpB fragment comprises or consists of amino acid sequence SEQ ID NO: 16.
**SEQ ID NO: 16**

   E-V-L-A-R-W-T-G-K-P-V

In a particular embodiment, the polypeptide or protein of the invention comprises the amino acid sequence SEQ ID NO: 6, 7, 8, 9, 10, 11, 12, 13, 14, 15 or 16 and has a length of at least 15 amino acids, preferably at least 20 amino acids.

In one embodiment, a ClpB fragment comprises or consists of an amino acid sequence presenting a sequence identity of at least 70% with amino acid sequence 538-548 of SEQ ID NO: 1 (EVLARWTGIPV, SEQ ID NO: 17), preferably of at least 80%, more preferably of at least 90%, 91%, 92%, 93%, 94%, 95%, 96%, 97%, 98%, 99% or more. In one embodiment, a ClpB fragment comprises or consists of the amino acid sequence SEQ ID NO: 17.

In one embodiment, a ClpB fragment comprises or consists of an amino acid sequence presenting a sequence identity of at least 70% with amino acid sequence 536-548 of SEQ ID NO: 1 (IAEVLARWTGIPV, SEQ ID NO: 18), preferably of at least 80%, more preferably of at least 90%, 91%, 92%, 93%, 94%, 95%, 96%, 97%, 98%, 99% or more. In one embodiment, a ClpB fragment comprises or consists of the amino acid sequence SEQ ID NO: 18.

In one embodiment, a ClpB fragment comprises or consists of an amino acid sequence presenting a sequence identity of at least 70% with amino acid sequence 534-548 of SEQ ID NO: 1 (AEIAEVLARWTGIPV, SEQ ID NO: 19), preferably of at least 80%, more preferably of at least 90%, 91%, 92%, 93%, 94%, 95%, 96%, 97%, 98%, 99% or more. In one embodiment, a ClpB fragment comprises or consists of the amino acid sequence SEQ ID NO: 19.

In one embodiment, a polypeptide or protein comprising or consisting of at least a fragment of a ClpB protein or variant thereof does not consist of the amino acid sequence of a ClpB protein. In other words, in one embodiment, the polypeptide or protein of the invention is not a full-length ClpB protein. Accordingly, in one embodiment, the amino acid sequence of the polypeptide or protein of the invention is not 100% identical to a ClpB sequence. In a particular embodiment, the amino acid sequence of the polypeptide or protein of the invention is not 100% identical to SEQ ID NO: 1.

In one embodiment, a polypeptide or protein comprising or consisting of at least a fragment of a ClpB protein or variant thereof does not consist of the amino acid sequence of α-MSH.

In one embodiment, the polypeptide or protein of the invention or variant thereof has homology with α-MSH (SEQ ID NO: 20). In one embodiment, the polypeptide or protein of the invention or variant thereof has conformational homology with α-MSH. In another embodiment, the polypeptide or protein of the invention or variant thereof has sequence homology with α-MSH. In another embodiment, the polypeptide or protein of the invention or variant thereof has conformational and sequence homology with α-MSH.

In one embodiment, the polypeptide or protein of the invention or variant thereof is recognized by an anti-α-MSH antibody. In other words, in one embodiment, the polypeptide or protein of the invention or variant thereof serves as an epitope for an anti-α-MSH antibody. In one embodiment, the polypeptide or protein of the invention or variant thereof serves as a conformational epitope for an anti-α-MSH antibody.

In one embodiment, a polypeptide or protein comprising or consisting of at least a fragment of a ClpB protein or variant thereof is a polypeptide derivative.

In one embodiment, the polypeptide or protein of the invention or variant thereof is modified by means well-known in the art, e.g., by the addition of one or more functional group such as a phosphate, acetate, lipid or carbohydrate group, and/or by the addition of one or more protecting group. For example, the polypeptide or protein of the invention or variant thereof can be modified by the addition of one or more functional groups such as phosphate, acetate, or various lipids and carbohydrates.

The polypeptide or protein of the invention or variant thereof described herein can be produced synthetically by chemical synthesis or enzymatic synthesis as it is well known in the art. Alternatively, nucleotide sequences encoding the polypeptide or protein of the invention or variant thereof of the invention can be introduced into a protein expression vector and produced in a suitable host organism (e.g., bacteria, insect cells, etc.), then purified. In one embodiment, the polypeptide or protein of the invention or variant thereof is obtained by a cloning method, such as, for example, using any production system known in the art, such as, for example, *E. coli,* yeast, baculovirus-insect cell, or mammalian cells such as HEK or CHO expression system.

An additional polypeptide ("tag") can be added on for the purpose of purifying or identifying or purifying the polypeptides. Protein tags make it possible, for example, for the polypeptides to be adsorbed, with high affinity, to a matrix, and for the matrix then to be washed stringently with suitable buffers without the complex being eluted to any significant extent, and for the adsorbed complex subsequently to be eluted selectively. Examples of protein tags which are known to the skilled person are a (His)₆ tag, a Myc tag, a FLAG tag, a hemagglutinin tag, a glutathione transferase (GST) tag, intein having an affinity chitin- binding tag or maltose-binding protein (MBP) tag. These protein tags can be located N- terminally, C -terminally and/or internally.

The present invention further relates to a polynucleotide or nucleic acid encoding a polypeptide or protein comprising or consisting of at least a fragment of the ClpB protein or variant thereof as described herein.

In one embodiment, the polynucleotide or nucleic acid of the invention is DNA. In another embodiment, the polynucleotide or nucleic acid of the invention is RNA, for example, in the form of messenger RNA (mRNA).

In one embodiment, the polynucleotide or nucleic acid of the present invention is single stranded. In another embodiment, the polynucleotide or nucleic acid of the present invention is double stranded.

Another object of the present invention is a vector encoding a polypeptide or protein comprising or consisting of at least a fragment of the ClpB protein or variant thereof as described herein, or comprising a polynucleotide encoding a polypeptide or protein comprising or consisting of at least a fragment of the ClpB protein or variant thereof as described herein.

Examples of vector include, but are not limited to, a plasmid, a bacteriophage, a virus, a cationic vesicle or any other type of vector.

In one embodiment, the vector of the invention is an expression vector.

Another object of the present invention is a composition comprising a polypeptide or protein or variant thereof as described herein, or a polynucleotide or nucleic acid as described herein, or a vector as described herein.

Another object of the present invention is a pharmaceutical composition comprising a polypeptide or protein or variant thereof as described herein, or a polynucleotide or nucleic acid as described herein, or a vector as described herein, and at least one pharmaceutically acceptable excipient.

Another object of the present invention is a medicament comprising a polypeptide or protein or variant thereof as described herein, or a polynucleotide or nucleic acid as described herein, or a vector as described herein.

In one embodiment, the pharmaceutical composition or the medicament of the invention comprises a therapeutically effective amount of the polypeptide, protein, polynucleotide or vector of the invention.

Another object of the present invention is a vaccine comprising a polypeptide or protein or variant thereof as described herein, or a polynucleotide or nucleic acid as described herein, or a vector as described herein.

In one embodiment, the vaccine according to the present invention is for use in the prevention of overweight and/or obesity-related diseases and disorders.

Another object of the present invention is a dietary supplement or protein dietary supplement comprising a polypeptide comprising a fragment of a ClpB protein as described herein, preferably a polypeptide comprising a ClpB fragment comprising the amino acid sequence SEQ ID NO:2.

Another object of the present invention is a food composition comprising a polypeptide comprising a fragment of a ClpB protein as described herein, preferably a polypeptide comprising a ClpB fragment comprising the amino acid sequence SEQ ID NO:2.

In one embodiment, the dietary supplement or food composition comprises or consists of about 1% to about 50% by weight, preferably from about 2% to about 25% by weight of the polypeptide comprising a fragment of a ClpB protein according to the invention.

In one embodiment, the dietary supplement or food composition comprises or consists of at least 1%, 2%, 3%, 4%, 5%, 6%, 7%, 8%, 9%, 10%, 15%, 20%, 25%, 30%, 40%, 50%, 60%, 70%, 80% by weight or more of the polypeptide comprising a fragment of a ClpB protein according to the invention.

In one embodiment, the dietary supplement or food composition of the present invention comprises at least one additional ingredient selected from among simple and/or complex carbohydrates, lipids, fibers, or minerals.

In one embodiment, the dietary supplement or food composition of the present invention comprises at least one essential amino acid. Preferably, said at least one essential amino acid is isolated or free, *i.e.,* not bonded in a protein chain.

In one embodiment, the at least one plant extract is obtained from at least one portion of the respective vegetable or plant. In particular, the vegetable or plant portion from which the at least one plant extract is obtained include, but are not limited to, leaves, bark, roots, rootstocks, stem, seeds and/or flowers, in accordance with the characteristics of the plant extract that one wishes to obtain or with the particular characteristics of the vegetable or plant, also in accordance with the plant portions in which the active ingredients, preferably the ClpB protein, fragment or variant thereof, which are desired to be present in the at least one plant extract, are contained.

In one embodiment, the at least one plant extract can be present in the form of a powder, micronized powder, lyophilized powder, wet powder, aqueous and non-aqueous solutions, suspensions in non-polar solvents such as resins or oils.

In one embodiment, the dietary supplement or food composition according to the present invention is in the form of anhydrous powder or powder containing water or moisture.

In one embodiment, the dietary supplement or food composition further comprises carriers or vehicles. "Carriers" or "vehicles" mean materials suitable for administration and include any such material known in the art such as, for example, any liquid, gel, solvent, liquid diluent, solubilizer, or the like, which is non-toxic and which does not interact with any components, in particular with the bacterial strain, of the composition in a deleterious manner. Examples of nutritionally acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

In one embodiment, the dietary supplement or food composition of the present invention comprises an amount of dietary fibers. Dietary fibers pass through the small intestine undigested by enzymes and functions as a natural bulking agent and laxative. Dietary fibers may be soluble or insoluble and in general a blend of the two types is preferred. Suitable sources of dietary fibers include soy, pea, oat, pectin, guar gum, gum Arabic, fructooligosaccharides, galacto-oligosaccharides, sialyl-lactose and oligosaccharides derived from animal milks. In some embodiments, the dietary fiber is selected among mannans. Mannans (such as glucomannans and galactomannans), such as guar gum, locust bean gum, konjac, and xanthan gum, are present in some plant cell walls. The glucomannans are generally comprised of (1-4)-β-linked glucose and mannose units, while the galactomannans are generally comprised of a (1-4)-β-mannan backbone substituted with single units of (1-6)-α-galactose. Many endospermic legumes, such as guar and locust bean, contain galactomannans in the endosperm during seed development. Glucomannans have also been found as a minor component of cereal grains.

In one embodiment, the dietary supplement or food composition of the present invention contains minerals and micronutrients such as trace elements and vitamins in accordance with the recommendations of Government bodies such as the USRDA. For example, the composition may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 µg Vitamin E.

In one embodiment, the dietary supplement or food composition of the present invention further comprises emulsifiers. Examples of food grade emulsifiers typically include diacetyl tartaric acid esters of mono- and di-glycerides, lecithin and mono- and di-glycerides. Similarly, suitable salts and stabilisers may be included.

In one embodiment, the dietary supplement or food composition according to the present invention can be used for the preparation of dietary food.

In one embodiment, the dietary supplement or food composition according to the present invention can be used for introduction into daily meals.

One aspect of the present invention relates to a method for treating or preventing overweight and/or obesity-related diseases and disorders in a subject in need thereof, comprising administering to the subject an effective amount of a polypeptide or protein comprising or consisting of at least a fragment of the ClpB protein or variant thereof as described hereinabove.

The present invention also relates to a polypeptide or protein comprising or consisting of at least a fragment of the ClpB protein or variant thereof, a polynucleotide, a vaccine, a pharmaceutical composition or a medicament as described hereinabove, for use for treating inflammation in a subject in need thereof.

Within the meaning of the invention, by "inflammation", it is meant, as defined in Dorland's Medical Dictionary, "a localized protective response, elicited by injury or destruction of tissues, which serves to destroy, dilute or wall off both the injurious agent and the injured tissue". It is characterized by fenestration of the microvasculature, leakage of the elements of blood into the interstitial spaces, and migration of leukocytes into the inflamed tissue. On a macroscopic level, this is usually accompanied by the familiar clinical signs of erythema, edema, hyperalgesia (tenderness), and pain.

In one embodiment, the polypeptide or protein, polynucleotide, vaccine, pharmaceutical composition or medicament according to the invention is for use in the treatment of inflammation, wherein said inflammation is selected from the group comprising obesity, obesity-related diseases or disorders, neuroinflammation, multiple sclerosis, atherosclerosis, allergies, ankylosing spondylitis, arthritis (osteoarthritis, rheumatoid arthritis, or psoriatic arthritis), asthma, graft versus host disease, Parkinson's disease, Alzheimer's disease, Crohn's disease, colitis, dermatitis, diverticulitis, fibromyalgia, hepatitis, irritable bowel syndrome, systemic lupus erythematous, nephritis, and ulcerative colitis.

In one embodiment, the inflammation of the invention is an acute inflammation. In another embodiment, the inflammation of the invention is a chronic inflammation.

In one embodiment, the polypeptide or protein, polynucleotide, vaccine, pharmaceutical composition or medicament according to the invention is for use in the treatment of inflammation, wherein said inflammation is selected from the group comprising or consisting of obesity, obesity-related diseases or disorders, neuroinflammation, multiple sclerosis, psoriasis, allergic rhinitis, osteoarthritis and neuroinflammatory diseases. In a particular embodiment, the polypeptide or protein, polynucleotide, vaccine, pharmaceutical composition or medicament according to the invention is for use in the treatment of inflammation, wherein said inflammation is selected from the group comprising or consisting of obesity, neuroinflammation, multiple sclerosis, psoriasis, allergic rhinitis, osteoarthritis and neuroinflammatory diseases.

An object of the present invention relates to a polypeptide or protein comprising or consisting of at least a fragment of the ClpB protein or variant thereof, polynucleotide, vaccine, pharmaceutical composition or medicament according to the invention for use in the treatment or prevention of obesity in a subject in need thereof.

Another object of the present invention relates to a method for treating or preventing obesity in a subject in need thereof, comprising administering to the subject an effective amount of a polypeptide or protein comprising or consisting of at least a fragment of the ClpB protein or variant thereof, polynucleotide, vaccine, pharmaceutical composition or medicament as described hereinabove.

One aspect of the present invention relates to a polypeptide or protein comprising or consisting of at least a fragment of the ClpB protein or variant thereof, polynucleotide, vaccine, pharmaceutical composition or medicament as described hereinabove, for use in the treatment or prevention of overweight and/or obesity-related diseases and disorders.

Overweight and/or obesity-related diseases and disorders include, but are not limited to, high blood pressure, diabetes (in particular, type 2 diabetes), glucose intolerance, insulin resistance, cardiovascular disease (such as atherosclerosis, coronary artery disease, narrowed arteries, angina, heart attack, blood clots), high cholesterol, fatty liver disease, hepatic steatosis, cholelithiasis, joint problems, osteoarthritis, orthopedic problems, impaired balance, skin conditions, sleep apnea, respiratory problems, asthma, heavy snoring, cancer (including breast, colon, gallbladder, uterus, colon and prostate cancers), metabolic syndrome, menstrual abnormalities and psychosocial effects.

One aspect of the present invention relates to a method of inducing satiation in a subject in need thereof comprising administering to the subject an effective amount of a polypeptide or protein, polynucleotide, vector, composition, pharmaceutical composition, medicament or vaccine according to the invention.

One aspect of the present invention relates to a polypeptide or protein, polynucleotide, vector, composition, pharmaceutical composition, medicament or vaccine according to the invention for use for inducing satiation in a subject in need thereof.

One aspect of the present invention concerns the non-therapeutic use of a polypeptide or protein, polynucleotide, vector, composition, dietary supplement or food composition according to the invention for inducing satiation in a subject.

One aspect of the present invention relates to a method of prolonging satiety in a subject in need thereof comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, medicament, vaccine, dietary supplement or food composition according to the invention for use for prolonging satiety in a subject in need thereof.

One aspect of the present invention concerns the non-therapeutic use of a polypeptide or protein, polynucleotide, vector, composition, dietary supplement or food composition according to the invention for prolonging satiety in a subject.

In one embodiment, the prolongation of satiety is of at least 1%, 2%, 3%, 4%, 5%, 6%, 7%, 8%, 9% or 10%. In one embodiment, the reduction of meal size is of at least 15%, 20% or 25%.

One aspect of the present invention relates to a method of reducing meal size in a subject in need thereof comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, pharmaceutical composition, medicament, vaccine, dietary supplement or food composition according to the invention for use for reducing meal size in a subject in need thereof.

One aspect of the present invention concerns the non-therapeutic use of a polypeptide or protein, polynucleotide, vector, composition, dietary supplement or food composition according to the invention for reducing meal size in a subject.

In one embodiment, the reduction of meal size is of at least 1%, 2%, 3%, 4%, 5%, 6%, 7%, 8%, 9% or 10%. In one embodiment, the reduction of meal size is of at least 15%, 20% or 25%.

One aspect of the present invention relates to a method of reducing food intake in a subject in need thereof comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, pharmaceutical composition, medicament, vaccine, dietary supplement or food composition according to the invention for use for reducing food intake in a subject in need thereof.

One aspect of the present invention concerns the non-therapeutic use of a polypeptide or protein, polynucleotide, vector, composition, dietary supplement or food composition according to the invention for reducing food intake in a subject.

In one embodiment, the reduction of food intake is of at least 1%, 2%, 3%, 4%, 5%, 6%, 7%, 8%, 9% or 10%. In one embodiment, the reduction of food intake is of at least 15%, 20% or 25%.

One aspect of the present invention also relates to a method of controlling, in particular reducing, weight gain in a subject in need thereof comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, pharmaceutical composition, medicament, vaccine, dietary supplement or food composition according to the invention for use for controlling, in particular reducing, weight gain in a subject in need thereof.

One aspect of the present invention concerns the non-therapeutic use of a polypeptide or protein, polynucleotide, vector, composition, dietary supplement or food composition according to the invention for controlling, in particular reducing, weight gain in a subject.

One further aspect of the present invention also relates to a method of stimulating weight loss in a subject in need thereof comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, pharmaceutical composition, medicament, vaccine, dietary supplement or food composition according to the invention for use for stimulating weight loss in a subject in need thereof.

One aspect of the present invention concerns the non-therapeutic use of a polypeptide or protein, polynucleotide, vector, composition, dietary supplement or food composition according to the invention for stimulating weight loss in a subject.

In one embodiment, the stimulation of weight loss is of at least 1%, 2%, 3%, 4%, 5%, 6%, 7%, 8%, 9% or 10%. In one embodiment, the stimulation of weight loss is of at least 15%, 20% or 25%.

One further aspect of the present invention also relates to a method of reducing weight in a subject in need thereof comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, pharmaceutical composition, medicament, vaccine, dietary supplement or food composition according to the invention for use for s reducing weight in a subject in need thereof.

One aspect of the present invention concerns the non-therapeutic use of a polypeptide or protein, polynucleotide, vector, composition, dietary supplement or food composition according to the invention for reducing weight in a subject.

In one embodiment, the reduction of weight is of at least 1%, 2%, 3%, 4%, 5%, 6%, 7%, 8%, 9% or 10%. In one embodiment, the reduction of weight is of at least 15%, 20% or 25%.

One aspect of the present invention relates to a method of reducing fat mass on lean mass ratio in a subject in need thereof, comprising administering to the subject an effective amount of a polypeptide or protein, polynucleotide, vector, composition, pharmaceutical composition, medicament, vaccine, dietary supplement or food composition according to the invention.

One aspect of the present invention relates to a polypeptide or protein, polynucleotide, vector, composition, pharmaceutical composition, medicament, vaccine, dietary supplement or food composition according to the invention for use for reducing fat mass on lean mass ratio in a subject in need thereof, in particular in an obese subject.

One aspect of the present invention concerns the non-therapeutic use of a polypeptide or protein, polynucleotide, vector, composition, dietary supplement or food composition according to the invention for reducing fat mass on lean mass ratio in a subject, in particular in a subject having normal weight or uncomplicated overweight.

In one embodiment, the reduction of fat mass on lean mass ratio is of at least 1%, 2%, 3%, 4%, 5%, 6%, 7%, 8%, 9% or 10%. In one embodiment, the s reduction of fat mass on lean mass ratio is of at least 15%, 20% or 25%.

In one embodiment, the use of a polypeptide or protein, polynucleotide, vector, composition, dietary supplement or food composition according to the invention is a cosmetic use.

In one embodiment, the subject is a female. In some embodiments, the subject is a male.

In one embodiment, the subject is a child, such as an individual aged less than 18 (in human years). In another embodiment, the subject is an adult, such as an individual aged 18 or more (in human years).

In one embodiment, the subject is obese. In one embodiment, the subject has a body mass index (BMI) above 30.

In one embodiment, the subject is moderately obese. In one embodiment, the subject has a BMI ranging from about 30 to about 35. In one embodiment, the subject is severely obese. In one embodiment, the subject has a BMI ranging from about 35 to about 40. In one embodiment, the subject is morbidly obese. In one embodiment, the subject has a BMI ranging from about 40 to about 50 or more.

In one embodiment, the subject is not obese. In one embodiment, the subject has a BMI below 30. In one embodiment, the subject is overweight. Accordingly, in one embodiment, the subject has a BMI ranging from about 25 to about 30.

In one embodiment, the subject is a healthy overweight subject. In one embodiment, the subject is a non-healthy overweight subject.

In one embodiment, the subject has a normal body weight. In one embodiment, the subject has a BMI ranging from about 18.5 and 25.

In one embodiment, the subject is under a slimming diet and/or wants to lose weight. In another embodiment, the subject is not under a slimming diet and/or does not want to lose weight.

In one embodiment, the subject is at risk of gaining weight. In some embodiments, the subject is at risk of accumulating excessive fat.

In one embodiment, the subject is at risk of developing overweight and/or obesity. In one embodiment, the subject is at risk of developing overweight and/or obesity-related diseases and disorders.

In one embodiment, the subject is a binge-eater. In one embodiment, the subject suffers from binge- or compulsive eating disorder.

In one embodiment, the polypeptide or protein, polynucleotide or vector according to the invention is administered to the subject in the form of a composition, pharmaceutical composition, medicament or vaccine. In one embodiment, the polypeptide or protein, polynucleotide or vector according to the invention is combined with pharmaceutically acceptable excipients, and optionally sustained-release matrices, such as biodegradable polymers, to form pharmaceutical compositions.

In one embodiment, the polypeptide or protein, polynucleotide, vector, composition, pharmaceutical composition, medicament or vaccine according to the invention is to be administered orally, by injection, topically, nasally, buccally, rectally, vaginaly, intratracheally, by endoscopy, transmucosally, or by percutaneous administration.

The disclosed polypeptides or proteins, polynucleotide, vectors, compositions, pharmaceutical compositions, medicaments or vaccines according to the invention can be delivered to the target cells in a variety of ways. The delivery mechanism chosen will depend in part on the type of cell targeted and whether the delivery is occurring for example *in vivo* or *in vitro.* The skilled artisan will be able to adapt the delivery of the polypeptides or nucleic acid sequences of the invention.

In one embodiment, the polypeptide or protein, polynucleotide, vector, composition, pharmaceutical composition, medicament or vaccine according to the invention is to be orally administered. Examples of formulations adapted to oral administration include, but are not limited to: solid forms, liquid forms and gels. Examples of solid forms adapted to oral administration include, but are not limited to, pill, tablet, capsule, soft gelatin capsule, hard gelatin capsule, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

In one embodiment, the polypeptide or protein, polynucleotide, vector, composition, pharmaceutical composition, medicament or vaccine according to the invention is to be administered by injection, preferably systemically injected. Examples of formulations adapted to systemic injections include, but are not limited to: liquid solutions or suspensions, solid forms suitable for solution in, or suspension in, liquid prior to injection. Examples of systemic injections include, but are not limited to, intravenous, subcutaneous, intramuscular, intradermal, intravitreal, and intraperitoneal injection, or perfusion. In another embodiment, when injected, the composition, the pharmaceutical composition or the medicament of the invention is sterile. Methods for obtaining a sterile pharmaceutical composition include, but are not limited to, GMP synthesis (GMP stands for "Good manufacturing practice").

In another embodiment, the polypeptide or protein, polynucleotide, vector, composition, pharmaceutical composition, medicament or vaccine according to the invention is to be topically administered. Examples of formulations adapted to topical administration include, but are not limited to, sticks, waxes, creams, lotions, ointments, balms, gels, masks, leave-on washes and/or the like.

In one embodiment of the invention, the ointment is an oleaginous ointment; an emulsified ointment such as, for example, oil-in-water or a water-in-oil ointment; or a water-soluble ointment, preferably is an oleaginous ointment.

In one embodiment of the invention, the oleaginous ointment uses bases such as, for example, plant and animal oils; plant and animal fats; waxes; vaseline, such as, for example, white vaseline or vaseline oil; and paraffin such as, for example, liquid paraffin or paraffin oil.

In another embodiment, the polypeptide or protein, polynucleotide, vector, composition, pharmaceutical composition, medicament or vaccine according to the invention can also be applied topically using a transdermal system, such as one of an acrylic-based polymer adhesive with a resinous crosslinking agent impregnated with the composition and laminated to an impermeable backing. Examples of formulations adapted to transdermal administration include, but are not limited to, ointment, paste, cream, film, balm, patch, such as, for example, transdermal patch, gel, liposomal forms and the like.

In one embodiment, the polypeptide or protein, polynucleotide, vector, composition, pharmaceutical composition, medicament or vaccine according to the invention can be administered topically as a transdermal patch, more particularly as a sustained-release transdermal patch. The transdermal patches can include any conventional form such as, for example, adhesive matrix, polymeric matrix, reservoir patch, matrix or monolithic-type laminated structure, and are generally comprised of one or more backing layers, adhesives, penetration enhancers, an optional rate controlling membrane and a release liner which is removed to expose the adhesives prior to application. Polymeric matrix patches also comprise a polymeric-matrix forming material. Suitable transdermal patches are well described in the art [see e.g., US patents No 5262165, 5948433, 6010715 and 6071531].

In one embodiment, the polypeptide or protein, polynucleotide, vector, composition, pharmaceutical composition, medicament or vaccine according to the invention is administered in a controlled-release, delayed-release, extended-release, long-acting-release, modified-release, sustained-release or timed-release form. Therefore, the polypeptide or protein, polynucleotide, vector, composition, pharmaceutical composition, medicament or vaccine according to the invention further comprise sustained-release matrices, such as biodegradable polymers.

In one embodiment, the dietary supplement or food composition according to the invention is to be orally administered. Examples of formulations adapted to oral administration include, but are not limited to: solid forms, liquid forms and gels. Examples of solid forms adapted to oral administration include, but are not limited to, pill, tablet, capsule, soft gelatin capsule, hard gelatin capsule, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

In one embodiment, the dietary supplement or food composition according to the invention is formulated as powders, for example, for mixing with consumable liquids such as milk, juice, water or consumable gels or syrups for mixing into other dietary liquids or foods. In one embodiment, the dietary supplement or food composition according to the invention is formulated with other foods or liquids to provide premeasured supplemental foods, such as single serving bars, for example. Flavorings, binders, protein, complex carbohydrates, and the like may be added as needed.

In one embodiment, the dietary supplement or food composition according to the invention is formulated using any pharmaceutically acceptable forms of the vitamins, minerals and other nutrients discussed above, including their salts. Preferred forms are calcium carbonate, magnesium hydroxide or magnesium sulfate, sodium tetraborate, cupric oxide, manganese sulfate, zinc sulfate, cholecalciferol, ferrous fumarate, pyridoxine hydrochloride, chromium picolinate, d-alphatocopherol acetate, and ascorbic acid.

In one embodiment, a therapeutically effective amount of the polypeptide or protein, polynucleotide, vector, composition, pharmaceutical composition, medicament, vaccine, dietary supplement or food composition according to the invention is administered at least once a day, twice a day, or at least three times a day. In another embodiment, a therapeutically effective amount of the polypeptide or protein, polynucleotide, vector, composition, pharmaceutical composition, medicament or vaccine according to the invention is administered every day, every two, three, four, five, six or seven days.

In another embodiment, a therapeutically effective amount of the polypeptide or protein, polynucleotide, vector, composition, pharmaceutical composition, medicament, vaccine, dietary supplement or food composition according to the invention is administered every week, twice a week, every two weeks, or once a month. In another embodiment, a therapeutically effective amount of the polypeptide or protein, polynucleotide, vector, composition, pharmaceutical composition, medicament or vaccine according to the invention is administered every month for a period at least 2, 3, 4, 5 or 6 months.

In another embodiment, a therapeutically effective amount of the polypeptide or protein, polynucleotide or vector according to the present invention ranges from about 1 µg to 5 g. In another embodiment, a therapeutically effective amount of the polypeptide or protein, polynucleotide or vector according to the present invention to be administered ranges from about 0.1 µg/kg to 1 g/kg.

In one embodiment, the method of the invention is for a chronic treatment, *i.e.,* the polypeptide or protein, polynucleotide, vector, composition, pharmaceutical composition, medicament, vaccine, dietary supplement or food composition according to the invention, is administered for a prolonged period of time, such as, for example, for at least about 1 week, 1 month, 1 year or more.

In another embodiment, the method of the invention is for an acute treatment, such as, for example, a treatment with only 1, 2 or 3 administrations of the polypeptide or protein, polynucleotide, vector, composition, pharmaceutical composition, medicament, vaccine, dietary supplement or food composition according to the invention.

In one embodiment, the administration of the polypeptide or protein, polynucleotide, vector, composition, pharmaceutical composition, medicament, vaccine, dietary supplement or food composition according to the invention is repeated, for example, 2 to 3 times a day, for one day or more and generally for a sustained period of at least 4 days, or even 4 to 15 weeks, with, where appropriate, one or more periods of interruption. In one embodiment, the polypeptide or protein, polynucleotide, vector, composition, pharmaceutical composition, medicament, vaccine, dietary supplement or food composition according to the invention is administered simultaneously or sequentially with one meal of the subject. In one embodiment, the polypeptide or protein, polynucleotide, vector, composition, pharmaceutical composition, medicament, vaccine, dietary supplement or food composition according to the invention is administered prior to the meal of the subject.

The present invention also relates to a kit comprising a polypeptide or protein, polynucleotide, composition, pharmaceutical composition, medicament, vaccine, dietary supplement or food composition according to the invention.

In one embodiment, the kit of the invention further comprises means to administer the polypeptide or protein, polynucleotide, pharmaceutical composition, medicament, vaccine, dietary supplement or food composition to a subject in need thereof.

Means to administer the polypeptide or protein, polynucleotide, pharmaceutical composition, medicament, vaccine, dietary supplement or food composition include, but are not limited to, syringes, needles, and other materials. In some embodiments, a means to administer the polypeptide or protein, polynucleotide, pharmaceutical composition, medicament, vaccine, dietary supplement or food composition include syringes pre-filled with the polypeptide or protein, polynucleotide, pharmaceutical composition, medicament or vaccine of the invention.

In one embodiment, the kit of the invention further comprises instructions for the administration of the polypeptide or protein, polynucleotide, pharmaceutical composition, medicament, vaccine, dietary supplement or food composition to said subject.

In one embodiment, the kit of the invention is for use for treating or preventing obesity in a subject in need thereof. In one embodiment, the kit of the invention is for use for treating or preventing overweight and/or obesity-related diseases or disorders in a subject in need thereof.

In one embodiment, the kit of the invention is used for preventing inducing satiation, prolonging satiety, reducing meal size, reducing food intake, controlling weight gain, reducing weight gain, stimulating weight loss, reducing weight and/or reducing fat mass on lean mass ratio in a subject in need thereof.

The invention will be further illustrated by the examples. However, these examples should not be interpreted in any way as limiting the scope of the present invention.

### BRIEF DESCRIPTION OF THE DRAWINGS

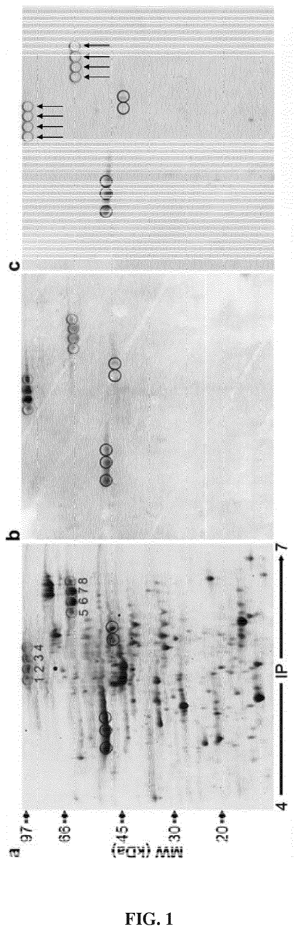

**Figure 1** is a photograph showing *E. coli* K12 cytoplasmic proteins separation using two-dimensional gel electrophoresis **(a)** and immunoblot with anti-α-MSH antibodies (**b** and **c**, preadsorbed by α-MSH) during proteomic identification of ClpB (spots 1, 2, 3 and 4). The spots 5, 6, 7 and 8 correspond to the protein GroEL (from Tennoune *et al.,* 2014).

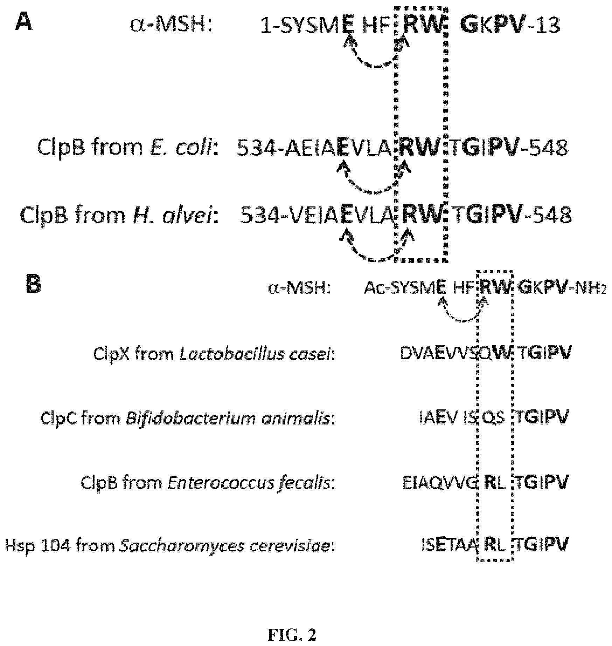

**Figure 2** is an alignment of sequences showing homology between the human α-MSH peptide and ClpB proteins from *E. coli* and *H. alvei* **(A),** and between α-MSH peptide and ClpB protein homologs from *L. casei, B. animalis, E. fecalis* and *S. cerevisiae* **(B)**.

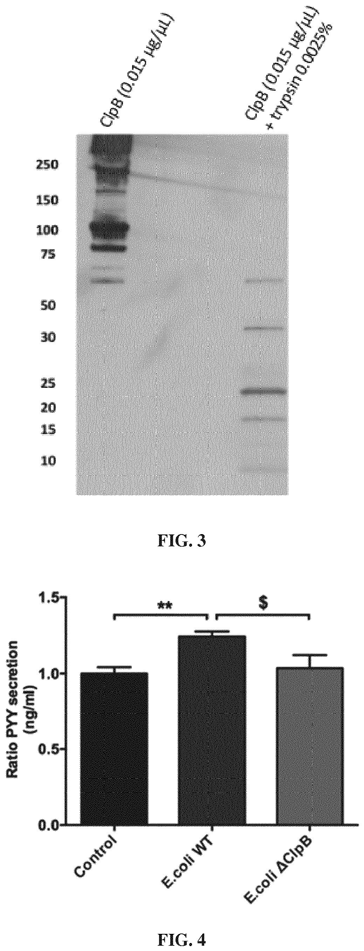

**Figure 3** is a photograph showing a Western Blot of ClpB proteins (left column) and fragments of ClpB proteins after treatment with trypsin 0.01% (right column), revealed with an anti-α-MSH antibody.
**Figure 4** is a graph showing PYY secretion of primary cultured intestinal cells incubated with total proteins from *E. coli* wild-type (middle column) and from *E. coli* ΔClpB (right column), both pre-incubated with trypsin 0.01%, normalized to total proteins from *E*. *coli* wild-type without pre-incubation (control, left column). **: unpaired t-test p=0.0078; $: unpaired t-test p=0.0571 (tendency).

### EXAMPLES

The present invention is further illustrated by the following examples.

### Example 1: Identification of bacterial protein with homology with α-MSH

Previous *in silico* analysis showed that several proteins of *E. coli* and some other microorganisms contain amino acid sequence homology with α-MSH, suggesting that they may be responsible for the production of a-MSH-reactive autoantibodies (Fetissov, in: Neuropsychiatric Disorders and Infection, Fatemi, S. H. (ed.), Taylor & Francis Books Ldt, 2004, pp 253-262; Fetissov et al., 2008. Nutrition. 24:348-359).

To find out which bacterial proteins may not be only theoretical but also functional α-MSH antigen-mimetics, a novel proteomic approach has been undertaken using *E. coli* strain K12. The novelty of this approach consisted in exploiting the well-known phenomenon of a so-called non-specific staining by primary antibodies in immunodetection protocols such as Western blot. In this approach, the "non-specific staining" of other proteins than the antigenic peptide used for antibody production was researched as a basis of a structural conformational homology between the α-MSH peptide and bacterial proteins.

### Materiel and methods

The proteomic approach reviewed here was used in the original study (Tennoune et al., 2014, Transl Psychiatry, 4:e458), and consisted in two-dimensional gel electrophoresis to separate total proteins of *E*. *coli* K12.

### Two-dimensional polyacrylamide gel electrophoresis

For two-dimensional (2D) polyacrylamide gel electrophoresis (PAGE), 400 µg of *E. coli* K12 protein extract were added to iso-electro focusing buffer (7 M urea, 2 M thiourea and 0.5% ampholytes, pH 4-7, 20 mM dithiothreitol, 2 mM tributyl phosphine, 2% CHAPS and 0.005% bromophenol blue) and solubilized for 60 min at room temperature with slight shaking. The first-dimensional gel separation was carried out using ReadyStrip IPG Strip (18 cm, pH 4-7 NL, Bio-Rad, Marnes-la-Coquette, France). After 24 h of passive rehydration of the strip with iso-electro focusing buffer, the protein sample was added to the strips through a loading cup placed at 1.5 cm from the cathode. Iso-electro focusing was performed with the Ettan IPGphor 3 System (GE Healthcare) in four steps (31 500 Vh): 500 V for 1 h, 1000 V gradient, 10 000 V gradient and 10 000 V for 2 h. After two equilibration steps with 2% dithiothreitol and 2.5% iodoacetamide, respectively, the second dimension, that is, a SDS-PAGE, (10% polyacrylamide gel, 20 cm × 18 cm × 1 mm) was performed on an Ettan Daltsix vertical electrophoresis system (GE Healthcare) with 12 mA per gel. After SDS-PAGE, the 2D gel was fixed for 2 h in 2% (vol:vol) orthophosphoric acid and in 50% (vol:vol) methanol at room temperature.

Gels were then rinsed with water, and the protein spots were visualized by CBB G-250 (Bio-Rad Laboratories, Hercules, CA, USA) staining (34% (vol:vol) methanol, 17% (wt:vol) ammonium sulfate, 2% (vol:vol) orthophosphoric acid and 0.66 g CBB G-250 per liter).

### Immunoblotting

Following 2D-PAGE, *E. coli* cytoplasmic proteins were transferred onto Hybond-ECL polyvinylidene difluoride membrane (GE Healthcare) via a dry transfer method (Trans Blot Cell, Bio-Rad) and a constant current of 0.8 mA.cm⁻² of the membrane size for 2 h. After transfer, membranes were blocked with 5% (wt:vol) milk (Regilait, Macon, France) in phosphatebuffered saline (PBS; 10 mmol.L⁻¹ Tris, pH 8, and 150 mmol.L⁻¹ NaCl) plus 0.05% (vol:vol) Tween 20. After washes, membranes were incubated overnight at 4°C with polyclonal rabbit anti-α-MSH IgG (1:1000, Peninsula Laboratories, San Carlos, CA, USA), followed by washes and incubation with polyclonal swine anti-rabbit horseradish peroxidase-conjugated Igs (1:3000; Dako, Trappes, France). Immunoblots were revealed by the ECL detection system (GE Healthcare) and were scanned with ImageScanner II (GE Healthcare) and digitalized with Labscan 6.00 software (GE Healthcare).

The same procedure was performed after adsorption of rabbit anti-α-MSH IgG with 10⁻⁶ M of α-MSH peptide (Bachem AG, Bubendorf, Switzerland) overnight at 4°C.

### Protein identification

The protein spots of interest were excised from CBB G-250-stained 2D gels using the Ettan Spot Picker (GE Healthcare), and automated in-gel digestion of proteins was performed on the Ettan Digester (GE Healthcare). Protein extracts were then resuspended in 10 µl of 5% (vol:vol) acetonitrile/0.1% (vol:vol) formic acid and then analyzed with a nano-LC1200 system coupled to a 6340 Ion Trap mass spectrometer equipped with a nanospray source and an HPLC-chip cube interface (Agilent Technologies, Courtaboeuf, France). In brief, peptides were enriched and desalted on a 40-nl RPC18 trap column and separated on a Zorbax (30-nm pore size, 5-µm particle size) C18 column (43 mm long × 75 µm inner diameter; Agilent Technologies). A 9-min linear gradient (3-80% acetonitrile in 0.1% formic acid) at a flow rate of 400 nl.min⁻¹ was used, and the eluent was analysed with an Ion Trap mass spectrometer. For protein identification, MS/MS peak lists were extracted and compared with the protein databases by using the MASCOT Daemon version 2.2.2 (Matrix Science, Boston, MA, USA) search engine. The searches were performed with the following specific parameters: enzyme specificity, trypsin; one missed cleavage permitted; no fixed modifications; variable modifications, methionine oxidation, cysteine carbamidomethylation, serine, tyrosine and threonine phosphorylation; monoisotopic; peptide charge, 2+ and 3+; mass tolerance for precursor ions, 1.5 Da; mass tolerance for fragmentations, 0.6 Da; electrospray ionization-Trap as instrument; taxonomy, *E. coli*; National Center for Biotechnology Information (NCBI) database (NCBI Nr 20120531 (18280215 sequences, 6265275233 residues); Bethesda, MD, USA). Protein hits were automatically validated if they satisfied one of the following criteria: identification with at least two top-ranking peptides (bold and red) each with a MASCOT score of 454 (Po0.01), or at least two top-ranking peptides each with a MASCOT score of 447 (Po0.05). To evaluate false positive rates, all the initial database searches were performed using the 'decoy' option of MASCOT. Results were considered relevant if the false positive rate never exceeded 1%.

### Results

Results of two-dimensional gel electrophoresis to separate total proteins of *E. coli* K12 are presented in **Figure 1a**. After transfer to a membrane, the presence of proteins containing α-MSH-like epitopes verified by application of polyclonal anti-α-MSH IgG revealed several positive spots (**Figure 1b**). However, application of anti-α-MSH IgG following their pre-adsorption with α-MSH peptide abolished only several positive spots (**Figure 1c**)**,** suggesting that only disappearing spots (arrows) should contain α-MSH-like epitopes. The mass spectrometry identification of the most strongly stained α-MSH-specifically stained spots revealed that they correspond to the caseinolytic protease B (ClpB) homologue, a 96 kDa protein of the heat shock family (spots 1, 2, 3, 4 of **Figure 1a****).**

### Example 2: Sequence alignment between CluB and a-MSH

The ClpB protein of *Enterobacteria* has been shown to stimulate production of α-MSH-cross-reactive antibodies. The inventors therefore hypothesized the presence of an α-MSH-like epitope in the ClpB protein of *Enterobacteria.*

Sequence alignment between ClpB and α-MSH revealed a discontinuous 6-amino acid sequence homology within the central part (amino acid residues 534-548 of SEQ ID NO: 1) of *E. coli* and *H. alvei* ClpB (**Figure 2A**). Such sequence was not predicted by an *in silico* analysis of homology to α-MSH in *E. coli* or *H. alvei* protein database using a search algorithm of α-MSH consecutive sequence homology. Moreover, *E. coli* and *H. alvei* proteins containing such consecutive sequences could not be identified by a proteomic approach (Fetissov et al., 2008. Transl Psychiatry, 2014, 4:e458), highlighting the difficulty for predicting the presence of peptide-like epitopes in large proteins having functional significance using *in silico* approach. Moreover, such sequence homology was not found in other bacteria or yeast, such as *L. casei, B. animalis, E. fecalis* and S. *cerevisiae* (**Figure 2B**).

α-MSH is a 13 amino acids acylated at the N-terminal peptide. The central part HFRW (amino acid residues 6 to 9 of SEQ ID NO: 2) represents the common pharmacophore of all central melanocortin (MC) system peptides, necessary for MC receptor activation with Arg(8) and Trp(9) most important amino acids, while both the N- and C-terminals differently participate in MCR binding (Hruby et al., 1987, J. Med. Chem. 30:2126-2130; Schiöth et al., European Journal of Pharmacology, 1998, 349:359-366; Haskell-Luevano et al., 2001, Journal of Medicinal Chemistry, 44:2247-2252). Presence of an acidic amino acid Glu(5) (amino acid residue 5 of SEQ ID NO: 2) may participate in α-MSH spatial conformation via forming a salt bridge with the basic Arg(8) (amino acid residue 8 of SEQ ID NO: 2). Such α-MSH folding forms a β-turn exposing the peptide core sequence necessary for MCRs activation (Li et al., 1999, European Journal of Biochemistry, 265:430-440).

ClpB sequence of *Enterobacteria* shares the following properties of the α-MSH peptide:
1. presence of consecutive Arg (R542 in SEQ ID NO: 1) and Trp (W543 in SEQ ID NO: 1) which are two critical amino acids in the α-MSH pharmacophore (R8W9 in SEQ ID NO: 2) necessary for activation of MC receptors;
2. presence of a negatively charged Glu (E538 in SEQ ID NO: 1) homologous to Glu (E5 in SEQ ID NO: 2) in α-MSH which can be important for the protein/peptide folding and exposing the central RW sequence at the β-turn;
3. presence of the GIPV545-548 (in SEQ ID NO: 1) sequence homology (75%) with GKPV10-13 (in SEQ ID NO: 2), *i.e.,* the C-terminal of α-MSH.

It is likely that combination of the 2 first properties is necessary for the unique functionality of the α-MSH-like epitope of ClpB (amino acid residues 534-548 of SEQ ID NO: 1), including both antigenic and α-MSH-like ligand interactions.

All commensal and pathogenic *Enterobacteriaceae* contained the identical α-MSH-like epitope of ClpB, suggesting the ability of bacteria belonging to this family to interfere with α-MSH signaling. Such bacteria include but are not limited to the genus of *Escherichia, Salmonella, Shigella, Klebsiella, Enterobacter, Citrobacter, Cronobacter* and *Hafnia.*

### Example 3: Effects of polypeptides of the invention on PYY and GLP-1 release

### Material & methods

### ClpB fragments identification

### ClpB fragmentation

First the fragmentation was performed on different conditions: ClpB diluted at 1/10 (from the stock at 5 mg/mL) and ClpB 1/10 with 0.0025% trypsin. Those different conditions were placed during 20 minutes at 37°C. After incubation, the fragmentation was immediately stopped by placing tubes on ice and western blot was performed.

### Western Blot

After fragmentation, Leamli solution without β-mercaptoethanol was added into tubes to perform western on 20% acrylamide SDS gel in Tris-Glycine buffer (BioRad, Hercules, USA) and transferred to a nitrocellulose membrane (GE Healthcare, Orsay, France). Then, the membrane was blocked for at least 1 h at room temperature with 5% (w/v) non-fat dry milk in TBS (10 mmol/L Tris, pH 8; 150 mmol/L NaCl) plus 0.05% (w/v) Tween 20. After blocking, the membrane was incubated overnight at 4°C with with anti-a-MSH antibody. The membrane was incubated overnight at 4°C with rabbit polyclonal anti-α-MSH antibodies (1:1000, Phoenix Pharmaceuticals). After three washes in a blocking solution of 5% (w/v) non-fat dry milk in TBS/0.05% Tween 20, membranes were incubated for 1 h with peroxidase-conjugated anti rabbit IgG (1:1000, SantaCruz Biotechnology). After three washes, the peroxidase reaction was revealed using the ECL detection kit (GE Healthcare). Protein bands were compared with the molecular weight standard (Precision Plus, BioRad) and films were scanned using ImageScanner III (GE Healthcare).

### PYY secretion experiment

### Cell culture

After euthanasia, rat colon was sampled and washed with fresh phosphate buffer saline (PBS). Intestinal tissue was then washed with L-15 medium (Leibovitz-15 medium; Sigma-Aldrich, Mo, US) maintaining a physiologic pH. Colic mucosa was scraped and digested with 0.4 mg/mL of collagenase IX (Psichas et al., 2015. Int J Obes (Lond). 39(3):424-9) in high glucose DMEM (Dulbecco's Modified Eagle Medium; Dominique Dutscher, France - supplemented with 5.5 mmol/L of L-glutamine, 100 U/mL of penicillin, 0.1 mg/mL of streptomycin and non-essential amino acids) at 37°C during 5-10 minutes. Cell suspensions were centrifuged at 750 rpm during 8 minutes and intestinal cells were suspended in the same supplemented DMEM medium in which 10% of fetal bovine serum was added. Cell suspensions were filtered at 100 µm (Merck Millipore, Mass, USA) and cultured into 24 wells plate coated with 1% Matrigel (Corning, NY, US). Finally, plates were incubated overnight at 37°C in a 95% O2/5% CO2 atmosphere.

### Cell lysate preparation

Previously, bacteria culture of wild type (WT) and ClpB depleted (ΔClpB) *E. coli* K12 were performed to proceed to total protein extraction. The protein extraction was performed in PBS containing 1% protease inhibitor (Sigma-Aldrich, Mo, US) by sonication during 30 seconds. Then, a centrifugation at 10000 rpm during 30 minutes at 4°C was realized. The supernatant containing proteins was then incubated with 0.0025% trypsin during 20 minutes at 37°C. After incubation, the fragmentation was immediately stopped by placing tubes on ice. The solution containing proteins was sampled and dosed to determine total protein concentration in each condition.

Concomitantly, intestinal cells were incubated in secretion buffer pH 7.4 (4.5 mM of KCl, 138 mM of NaCl, 4.2 mM of NaHCO3, 1.2mM de NaH2PO4, 2.6 mM de CaCl2, 1.2 Mm de MgCl2 and 10 mM of HEPES). Then cells were incubated during 20 minutes at 37°C in secretion buffer containing trypsinized fragments of proteins from *E. coli* K12 WT and *E. coli* K12 ΔClpB at 0.015 µg/µL (Breton et al., 2015. International Journal of Eating Disorders. 49:805-808; n=4 for each conditions). As a control, cells were also incubated in PBS.

After incubation, supernatants were sampled, centrifuged (10000 rpm during 3 minutes) and immediately stored at -80°C. Then cells were treated with a lysis buffer (50 mmol/L Tris-HCl, 150 mmol/L NaCl, 1% IGEPAL-CA 630, 0.5% desoxycholic acid and protease inhibitor cocktail without EDTA) to extract intracellular peptides. Cell lysates were immediately frozen at -80°C towards PYY measurements.

### PYY dosage

PYY dosage was performed on cell medium and cell lysates to measure PYY liberation (in the medium), production (within the lysates) and the total PYY relative production (medium and lysates). This dosage was realized using Fluorescent Immunoassay Kit® (Phoenix Pharmaceuticals, Inc.) according to the manufacturer instructions.

Briefly, after all reagent reconstitution, 50 µl of 1x assay buffer into 2 wells as total binding, 50 µl of prepared peptide standards (in duplicate), 50 µl of rehydrated positive control (in duplicate) and 50 µl of prepared samples (in duplicate) were charged onto the immunoplate. Then, 25 µl of rehydrated primary antibody and 25 µl of rehydrated biotinylated peptide were added into each well except the blank well. The microplate was incubated for 2 hours at room temperature (20-23°C) under orbital shaking at 300-400 rpm. After incubation, each well was washed four times with 350 µl of 1x assay buffer and 100 µl of SA-HRP antibody solution previously prepared by diluting 12 µl of SA-HRP into 12 ml of 1x assay buffer was added. The immunoplate was incubated again for 1 hour at room temperature (20-23°C) under orbital shaking at 300-400 rpm. After incubation, each well was washed in the same way as before and 100 µl of TMB substrate solution were added. The plate was incubated and protected from the light for 1 hour at room temperature (20-23°C) under orbital shaking at 300-400 rpm.

At the end, 100 µl 2N HCl were added into each well to stop the reaction (the colour in the well changed from blue to yellow) and the immunoplate was read onto a microtiter plate reader. Absorbance in optical density was read at 450 nm.

### Results

### ClpB fragments identification

The western blot performed on *E. coli* proteins pre-incubated with trypsin showed several bands when revealed with anti-α-MSH antibodies (Figure 3). Therefore, the step of trypsinisation produces several fragments of ClpB. In particular, a ∼25 kDa seems to be produced in larger amounts than other fragments.

Moreover, the western blot showed that no full-length ClpB is present in the solution after trypsinisation.

### PYY release

The same *E. coli* proteins pre-incubated with trypsin producing ClpB fragments as above were used for experiments on primary cultured intestinal cell from rat colon. Results show an increase of PYY release compared to control **(****Figure 4****).** On the contrary, no effect was observed with the ClpB depleted strain **(****Figure 4****).**

These results demonstrate that fragments of ClpB leads to a more efficient release of PYY, thereby demonstrating an activation of the MC4R (see Panaro et al., 2014. Cell Metab. 20(6):1018-1029).

## Claims

1. A polypeptide or protein of at least 15 amino acids comprising a fragment of a ClpB protein or variant thereof, comprising a negatively charged residue and two consecutive Arg and Trp residues, wherein said polypeptide or protein is not a full-length ClpB protein.

2. The polypeptide or protein according to claim **1,** wherein said polypeptide or protein comprises the amino acid sequence SEQ ID NO: 2.

3. The polypeptide or protein according to claim **1** or **2,** wherein said polypeptide or protein comprises the amino acid sequence SEQ ID NO: 3.

4. The polypeptide or protein according to any one of claims **1** to **3,** wherein said polypeptide or protein comprises the amino acid sequence SEQ ID NO: 4.

5. The polypeptide or protein according to any one of claims **1** to **4,** further comprising a sequence having at least 75% sequence homology with the sequence GKPV.

6. The polypeptide or protein according to any one of claims **1** to **5,** wherein said polypeptide or protein comprises the amino acid sequence SEQ ID NO: 6.

7. A composition comprising the polypeptide or protein according to any one of claims **1** to **6.**

8. A pharmaceutical composition comprising the polypeptide or protein according to any one of claims **1** to **6** and at least one pharmaceutically acceptable excipient.

9. A medicament comprising the polypeptide or protein according to any one of claims **1** to **6.**

10. A dietary supplement comprising the polypeptide or protein according to any one of claims **1** to **6.**

11. A food composition comprising the polypeptide or protein according to any one of claims **1** to **6.**

12. The polypeptide or protein according to any one of claims **1** to **6,** the pharmaceutical composition according to claim **8,** the medicament according to claim **9,** for use in the treatment or prevention of obesity, overweight and/or obesity-related diseases and disorders in a subject in need thereof.

13. Use of the polypeptide or protein according to any one of claims **1** to **6,** the dietary supplement according to claim **10** or the food composition according to claim 11, for reducing weight in a subject.

14. The polypeptide or protein, the pharmaceutical composition, the medicament, the dietary supplement or the food composition for use according to claim **12** or **13,** wherein the subject is not obese.

15. A kit comprising a polypeptide or protein according to any one of claims **1** to **6,** a composition according to claim **7,** a pharmaceutical composition according to claim **8,** a medicament according to claim **9,** a dietary supplement according to claim **10** or a food composition according to claim **11.**
